# EUROPEAN PATENT SPECIFICATION

(11) **EP 4 308 168 B1**
(45) Date of publication and mention of the grant of the patent: **20.05.2026**
(21) Application number: 22711109.3
(22) Date of filing: 16.03.2022
(51) Int. Cl.: A61K 47/68, A61P 35/00, C07C 51/15, C07C 51/367, C07C 51/64, C07C 269/04, C07D 207/46, C07K 16/30

(54) **METHODS FOR PREPARING CYCLOOCTENES AND CONJUGATES THEREOF**
VERFAHREN ZUR HERSTELLUNG VON CYCLOOCTENEN UND KONJUGATEN DAVON
PROCÉDÉS DE PRÉPARATION DE CYCLOOCTÈNES ET DE CONJUGUÉS DE CEUX-CI

(30) Priority: 16.03.2021 EP 21162953
(43) Date of publication of application: 24.01.2024
(73) Proprietor: Tagworks Pharmaceuticals B.V., 6525 ED Nijmegen (NL)
(72) Inventor: TEN HOEVE, Wolter, 6525 ED Nijmegen (NL); ROBILLARD, Marc Stefan, 6525 ED Nijmegen (NL); FERRER, Catalina, 6525 ED Nijmegen (NL); KLEIJN, Laurens Henri Johan, 6525 ED Nijmegen (NL); VERSTEEGEN, Ronny Mathieu, 6525 ED Nijmegen (NL)
(74) Representative: V.O.
(86) International application number: PCT/NL2022/050142
(87) International publication number: WO 2022/197182

(56) References cited:
- WO-A1-2020/256546
- RAFFAELLA ROSSIN ET AL: "Triggered Drug Release from an Antibody-Drug Conjugate Using Fast "Click-to-Release" Chemistry in Mice", BIOCONJUGATE CHEMISTRY, vol. 27, no. 7, 15 June 2016 (2016-06-15), US, pages 1697 - 1706, XP055404901, ISSN: 1043-1802, DOI: 10.1021/acs.bioconjchem.6b00231

## Description

### FIELD OF THE INVENTION

The present disclosure relates to methods for preparing cyclooctenes and conjugates thereof.

### BACKGROUND

In the art of bioorthogonal chemistry cyclooctenes are widely used. Examples of synthetic routes towards cyclooctenes suitable for bioorthogonal chemistry are provided by WO2020256546 and WO2019212357.

However, the synthesis can be cumbersome. For example, the synthesis of a cyclooctene to be used in bioorthogonal chemistry typically contains a large number of steps. In addition, it is difficult to prepare and/or isolate different stereoisomers if desired. Moreover, in some steps it is desired to perform regioselective reactions, which are not always readily available.

It is the aim of the invention to provide methods for the synthesis of cyclooctenes that overcome one or more of these problems.

### BRIEF DESCRIPTION OF THE FIGURES

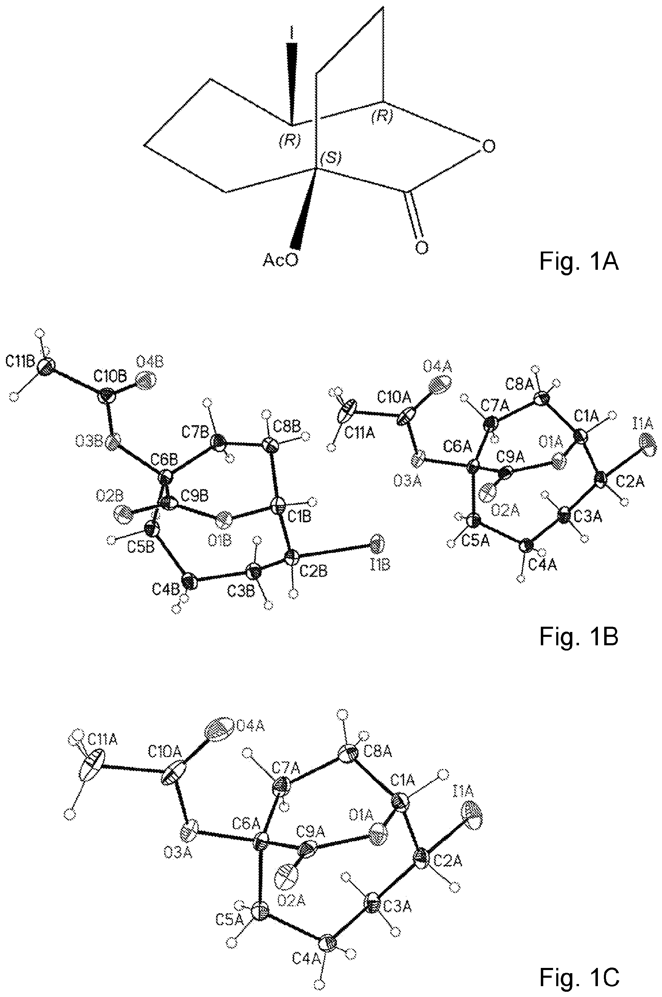

Figure 1A depicts the determined absolute configuration of compound 6A.
Figure 1B depicts an ORTEP-plot representation (50%) showing the two independent molecules present in the asymmetric unit of the structure of sample 6A.
Figure 1C depicts an ORTEP-plot representation (50%) showing the structure of compound 6A.

### DETAILED DESCRIPTION OF THE INVENTION

The invention, in a broad sense, is based on the judicious insight that one or more of the abovementioned desires is met by the methods as disclosed herein.

The invention relates to a method for regioselectively modifying a compound according to Formula (3) at the R² position, wherein said method comprises the step of:
contacting said compound according to Formula (3) or a salt thereof with a compound comprising a secondary amine;
wherein Formula (3) is: 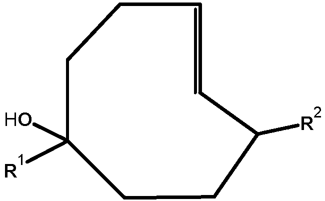
, wherein R¹ is selected from the group consisting of -C(O)O-N-succinimidyl, -C(O)O-pentafluorophenyl, -C(O)O-tetrafluorophenyl, -C(O)O-4-nitrophenyl, and -C(O)Cl; and wherein R² is selected from the group consisting of -OC(O)O-N-succinimidyl, -OC(O)O-pentafluorophenyl, -OC(O)O-tetrafluorophenyl, -OC(O)O-4-nitrophenyl, and -OC(O)Cl.

Without wishing to be bound by theory, the inventors have found that despite the relatively small difference in reactivity between positions R¹ and R², the method of the invention results in regioselective modification of the compound of Formula (3) at the R² position.

Preferably, the method for regioselectively modifying a compound according to Formula (3) comprises the steps of:
a) dissolving said compound according to Formula (3) or a salt thereof, to form a solution; and
b) contacting said solution with a compound comprising a secondary amine.

Compounds comprising secondary amines are used according to the invention, since the inventors found that these typically provide more steric hindrance than primary amines, and that this surprisingly leads to a more regioselective reaction. For the compound comprising a secondary amine, it is preferred that said compound does not contain primary amines. More preferably, said compound does not comprise other nucleophilic groups other than one or more secondary amines, preferably at most five secondary amines, more preferably at most three secondary amines, and most preferably at most one secondary amine. "Other nucleophilic groups" are for example thiolate groups.

Preferred compounds comprising a secondary amine are proteins and peptides, because typically the primary amines in these compounds are sterically hindered to a certain extent.

Preferably, the compound comprising a secondary amine is a drug or a protein, for example an antibody. Preferably, the compound comprising a secondary amine is monomethyl auristatin E. Preferably, compound 14 is provided in one of its enantiomerically enriched forms **14-E^{I}** and **14-E^{II}** as disclosed herein.

Preferably, the compound of Formula (3) is provided as a compound of Formula (3a) or as a compound of Formula (3b), or a mixture thereof, preferably a racemic mixture: 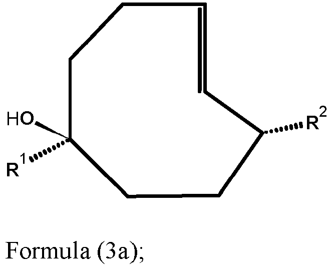 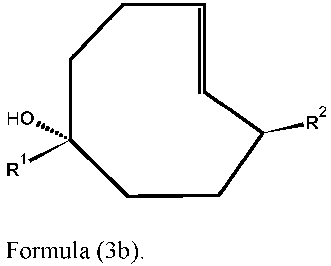 For ease of synthesis, the compound of Formula (3) is preferably provided as a mixture of a compound according to Formula (3a) and a compound according to Formula (3b), preferably a racemic mixture.
For compounds according to any one Formulae (3), (3a), and (3b), R¹ is selected from the group consisting of -C(O)O-N-succinimidyl, -C(O)O-pentafluorophenyl, -C(O)O-tetrafluorophenyl, -C(O)O-4-nitrophenyl, and -C(O)Cl. More preferably, R¹ is -C(O)O-*N-*succinimidyl, or -C(O)O-pentafluorophenyl. The inventors have found that the best results are obtained with these active esters.

For compounds according to any one Formulae (3), (3a), and (3b), R² is selected from the group consisting of -OC(O)O-N-succinimidyl, -OC(O)O-pentafluorophenyl, -OC(O)O-tetrafluorophenyl, -OC(O)O-4-nitrophenyl, and -OC(O)Cl. More preferably, R² is -OC(O)ON-succinimidyl, or -OC(O)O-pentafluorophenyl. The inventors have found that the best results are obtained with these active carbonates.

In preferred embodiments, the leaving groups of R¹ and R² are the same, e.g. both are -O-N-succinimidyl or both are -O-pentafluorophenyl. These embodiments are preferred, since the compounds of Formula (3) are easier to prepare when said leaving groups are the same. Therefore, in preferred embodiments R¹ is -C(O)O-N-succinimidyl, and R² is -OC(O)O-*N-*succinimidyl. In other preferred embodiments, R¹ is -C(O)O-pentafluorophenyl, and R² is - OC(O)O-pentafluorophenyl.

Preferably, the compound according to Formula (3) and the compound comprising a secondary amine are contacted in a molar ratio in a range of from 0.5:1 to 2:1, preferably in the a range of 0.6:1 to 1.5:1, more preferably in a range of from 0.75:1 to 1:1, and most preferably in stoichiometric amounts, *i.e.* 1:1.

When using the compound comprising a secondary amine the temperature of the reaction is not critical. For the sake of convenience, the regioselective modification is in that case preferably carried out in a range of from 10°C to 40 °C, more preferably in a range of from 15°C to 30 °C, most preferably in a range of from 20°C to 25 °C.

In other preferred embodiments, the regioselective modification is carried out at a temperature below room temperature, preferably in the range of from -80°C to 10 °C, more preferably in the range of from -40°C to 5 °C, even more preferably in a range of from -20°C to 0 °C, most preferably in a range of from -15°C to -5 °C.

Preferably, the method for regioselectively modifying a compound according to Formula (3) comprises the step of contacting said compound according to Formula (3) or a salt thereof with a compound comprising a secondary amine, and with a solvent. The order in which the three components are contacted is generally not important, but the inventors believe that the best results are obtained when the compound according to Formula (3) is first dissolved in the solvent, after which the solution is contacted with the compound comprising a secondary amine. More preferably, the compound comprising a secondary amine is added to the solution comprising the compound according to Formula (3).

In the method for regioselectively modifying a compound according to Formula (3) said compound can in principle be dissolved in any suitable solvent. The skilled person is readily capable of selecting a suitable solvent, for example by contacting said compound with a solvent. Preferably, the solvent is an organic solvent, more preferably the solvent is acetonitrile.

Upon contacting the compound of Formula (3) with the compound comprising a secondary amine and a solvent, the resulting solution is typically mixed, for example by stirring. The reaction time may vary depending on the reaction rates, especially the difference between reaction rates at the R¹ and the R² position in the compound of Formula (3). The skilled person is well aware of methods to determine the desired reaction time, for example by monitoring the reaction using analytical methods such as HPLC-MS and/or NMR. Typically, the reaction time is less than 1 hour, preferably less than 30 minutes, more preferably less than 15 minutes, most preferably less than 10 minutes. In general, the reaction time is at least 1 minute. The reaction can be quenched by using standard techniques, such as dilution, separation, cooling, and combinations thereof. The main product of the reaction, viz. the regioselectively modified compound, can be isolated using standard organic chemistry techniques known to the skilled person.

Preferably, the method for regioselectively modifying a compound according to Formula (3) is a method for the regioselectively modifying compound 14 wherein said method comprises the steps of:
a) dissolving compound 14 or a salt thereof, to form a solution;
b) contacting said solution with a compound comprising a secondary amine;
wherein compound 14 is provided as a racemic mixture of **14-E^{I}** and **14-E^{II}** as disclosed herein, or as one of the enantiomerically enriched forms **14-E^{I}** and **14-E^{II}** as disclosed herein.

### Definitions

The present invention will further be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where an indefinite or definite article is used when referring to a singular noun e.g. "a" or "an", "the", this includes a plural of that noun unless something else is specifically stated.

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

Thus, the scope of the expression "a device comprising means A and B" should not be limited to devices consisting only of components A and B. It means that with respect to the present invention, the only relevant components of the device are A and B.

The compounds disclosed in this description and in the claims may comprise one or more asymmetric centers, and different diastereomers and/or enantiomers may exist of the compounds. The description of any compound in this description and in the claims is meant to include all diastereomers, and mixtures thereof, unless stated otherwise. Thus, it will be understood that if herein a specific stereoisomer is indicated, the compound is limited to this specific stereoisomer.

In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer, unless stated otherwise.

For the sake of clarity, when compounds are labeled herein as Enantiomer A or Enantiomer B (e.g. compound **4B** herein), of as **E^{I}** or **E^{II}** (for example **4-E^{I}**), then the compound is limited to that specific enantiomer. Likewise if a compound is labeled as the axial isomer then that compound does not comprise the equatorial isomer.

The term "enantiomerically enriched" as used herein, refers to a composition of a chiral substance whose enantiomeric ratio is greater than 50:50. In certain embodiments , the enantiomerically enriched composition has a % ee of greater than 30%, greater than 40%, greater than 50%, greater than 60%, greater than 70, greater than 80, greater than 90%, greater than 95%, greater than 97%, greater than 99%, or 30%, or 40%, or 50%, or 60%, or 70, or 80, or 90%, or 95%, or 97%, or 98%, or 99%.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer, unless stated otherwise.

The compounds disclosed in this description and in the claims may further exist as exo and endo diastereomers. Unless stated otherwise, the description of any compound in the description and in the claims is meant to include both the individual exo and the individual endo diastereomers of a compound, as well as mixtures thereof. When the structure of a compound is depicted as a specific endo or exo diastereomer, it is to be understood that the invention of the present application is not limited to that specific endo or exo diastereomer, unless stated otherwise.

The compounds disclosed in this description and in the claims may further exist as *anti* and *syn* diastereomers. Unless stated otherwise, the description of any compound in the description and in the claims is meant to include both the individual *anti* and the individual *syn* diastereomers of a compound, as well as mixtures thereof. When the structure of a compound is depicted as a specific *syn* or *anti* diastereomer, it is to be understood that the invention of the present application is not limited to that specific *syn* or *anti* diastereomer, unless stated otherwise.

Unless stated otherwise, the compounds of the invention and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

In several formulae, groups or substituents are indicated with reference to letters such as "A", "B", "X", "Y", and various (numbered) "R" groups. In addition, the number of repeating units may be referred to with a letter, e.g. n in -(CH₂)ₙ-. The definitions of these letters are to be read with reference to each formula, i.e. in different formulae these letters, each independently, can have different meanings unless indicated otherwise.

The term "salt thereof means a compound formed when an acidic proton, typically a proton of an acid, is replaced by a cation, such as a metal cation or an organic cation and the like. The term "salt thereof also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted" salt means a salt that is acceptable for administration to a patient, such as a mammal (salts with counter-ions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

It should be noted that, depending on the choice of nomenclature, the TCO dienophile may also be denoted E-cyclooctene. With reference to the conventional nomenclature, it will be understood that, as a result of substitution on the cyclooctene ring, depending on the location and molecular weight of the substituent, the same cyclooctene isomer may formally become denoted as a Z-isomer. In the present invention, any substituted variants of the invention, whether or not formally "E" or "Z," or "cis" or "trans" isomers, will be considered derivatives of unsubstituted trans-cyclooctene, or unsubstituted E-cyclooctene. The terms "trans-cyclooctene" (TCO) as well as E-cyclooctene are used interchangeably and are maintained for all dienophiles according to the present invention, also in the event that substituents would formally require the opposite nomenclature. I.e., the invention relates to cyclooctene in which carbon atoms 1 and 6 as numbered below in Formula 2 are in the E (*entgegen*) or *trans* position. 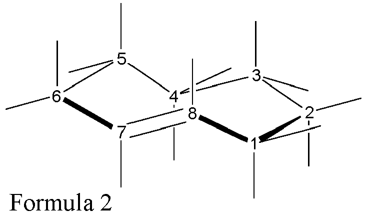 The TCO may consist of multiple isomers, also comprising the equatorial vs. axial positioning of substituents, such as R₄₈, on the TCO. In this respect, reference is made to Whitham et al. J. Chem. Soc. (C), 1971, 883-896, describing the synthesis and characterization of the equatorial and axial isomers of trans-cyclo-oct-2-en-ol, identified as (1RS, 2RS) and (1SR, 2RS), respectively. In these isomers the OH substituent is either in the equatorial or axial position. In a preferred embodiment, for TCO structures where the R₄₈ can be either in the axial or the equatorial position, the R₄₈ is in the axial position.

When in the following text reference is made to axial or equatorial isomers this pertains to the axial resp. equatorial positioning of the allylic hydroxyl or derived groups (i.e. R48).

R₄₈ as used herein is as defined in WO2020256546, in particular in claim 1 and on page 46 thereof. R₄₇ as used herein is as defined in WO2020256546, in particular in claim 1 on pages 63-65 thereof. Construct A or C^{A} as used herein is as defined in WO2020256546, in particular on pages 75-77, and 232-234 thereof. Construct B or C^{B} as used herein is as defined in WO2020256546, in particular on pages 75-77, and 232-234 thereof.

The invention is hereinafter illustrated with reference to the following, non-limiting, examples.

### EXAMPLES

### Example 1: Materials and methods

Unless indicated otherwise, all reagents, chemicals, materials and solvents were obtained from commercial sources, and were used as received.

NMR chemical shifts are reported in ppm downfield from TMS at 25°C. Abbreviations used for splitting patterns are s=singlet, t=triplet, q=quartet, m=multiplet and br=broad. Reverse phase (RP) medium pressure liquid column chromatograpy was performed on a Biotage Isolera One MPLC system using a GracePure C₁₈ RP column (40 gram), and acetonitrile / water mixtures (containing 0.1 v/v% formic acid) as the eluent. HPLC-MS/PDA was performed using a Shimadzu LC-20 AD XR series HPLC coupled to a diode array detector (Shimadzu SPD-M20A prominence) and an Ion-Trap (LCQ Fleet, Thermo Scientific) MS-detector, employing a Phenomenex Kinetex 5 µm EVO C18 column using an injection volume of 1-5 µL, a flow rate of 0.3 mL/min and typically a gradient (5% to 100% in 5 min, held at 100% for 1 min) of acetonitrile in H₂O (both containing 0.1 v/v% formic acid) at 20°C. Preparative RP-HPLC (acetonitrile / H₂O with 0.1 v/v% formic acid) was performed using a Shimadzu SCL-10A VP coupled to two Shimadzu LC-8A pumps and a Shimadzu SPD-10AV VP UV-vis detector on a Phenomenex Gemini 5µ C₁₈ 110A column. Size exclusion chromatography (SEC) was performed on an Akta system equipped with a Superdex 200 column. HPLC-QTOF-MS analysis was performed on a Waters Acquity UPLC system equipped with a Sample Manager and a Xevo G2 Quadrupole Time of Flight (QTOF) detector, applying Zspray lockspray ionisation. Mass Lynx v4.1 software was used. SDS polyacrylamide gel electrophoresis (SDS-PAGE) was performed on a Mini-PROTEAN Tetra Cell system using 4-15% precast Mini-PROTEAN TGX gels and Precision Plus Protein All Blue Prestained protein standards (Bio-Rad). The radioactivity distribution on TLC plates and SDS-PAGE gels was monitored with a Typhoon FLA 7000 phosphor imager (GE Healthcare Life Science) using the AIDA software.

### Example 2: Synthesis to bis-NHS TCO 14 (not claimed)

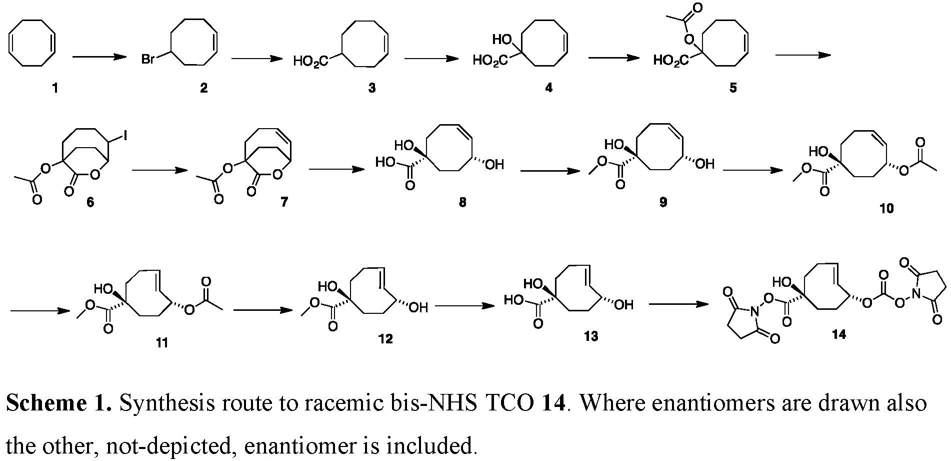

### Compound 2

Hydrogen bromide 33 % (893.7 g) in acetic acid (3.64 mol) was cooled in ice. Cyclooctadiene (548.6 g, 5.07 mol) was added in 90 min at 3 - 5°C with magnetic stirring. An additional amount of 350 g 33% hydrogen bromide in acetic acid was added in 1 h at 3 - 7°C. The mixture was stirred for 22 h. Water (400 mL) was added to the homogeneous solution, the layers were separated (the lower layer being the product layer) and the upper layer was extracted with 2 x 200 mL pentane. The product layer was stirred with 200 mL water and 84 g sodium bicarbonate was added in portions. The layers were separated and the water layer was extracted with the successive pentane layers. The successive organic layers were dried and then successively poured on a 600 g silica column, which was prepared with heptane. Pentane (200 mL) was first brought on the column and after that the successive product fractions, each being brought on the column when the previous had withdrawn into the column. Further elution was done with heptane to give 890 gram of crude **2** after rotary evaporation at 60°C, 40 mbar, which was used as such in the next step.

### Compound 3

Magnesium (66.6 g, 2.740 mol) was stirred magnetically with 380 mL THF. A 10 mL portion of **2** (total 491.3 g) was added, and when the reaction started another 800 mL THF was added. The remainder of **2** (in 320 mL THF) was added at 30 - 48°C in 2 h with cooling with an ice-bath. After the addition, another 250 mL THF was added, bringing the total THF to 1750 mL. After the addition, the mixture was brought to 60°C in 1 h, and kept at 60°C for 2 h. The blackish solution (small amount of magnesium present) was then cooled (forming a suspension) and carbon dioxide (from 850 g Dry Ice) was bubbled through. The temperature gradually rose to 38°C and the Grignard gradually dissolved. The thin suspension was stirred overnight. It was then poured onto 300 g Dry Ice in a large beaker. The supernatant was decanted and rotary evaporated at 60°C in order to remove ca. 850 mL THF. Water (300 mL) was added to the residue in the beaker and the mixture was filtered over a plug of cotton wool in order to remove the unreacted magnesium. The filtrate was then mixed with the remainder in the evaporation flask, followed by 500 mL heptane. The layers were separated and the upper layer was extracted with 3 x 100 mL water. The successive aqueous layers were extracted with 250 mL heptane. The aqueous layers were then combined, 500 mL toluene was added and the mixture was acidified with 160 mL 37% hydrochloric acid. The layers were separated and the lower layer was extracted with 400 mL, 250 mL and 100 mL toluene. Drying and rotary evaporation gave the acid **3.** In a similar reaction, the remaining 400 g of the bromide of the previous experiment had been converted into the acid, for a total yield of 385.0 g, after being put under high vacuum at 50°C for 1 h (2.497 mol, 49% based on cyclooctadiene). NMR showed a trace of toluene. The product was used as such in the next step.

Advantageously, the direct transformation of **2** to **3** eliminates one reaction step compared to the method in WO2020256546.

### Compound 4

A solution of 203.0 g diisopropylamine (2.01 mol) in 750 mL THF was cooled and 850 mL 2.5 N n-butyllithium (2.125 mol) was added in 1.5 h at -50 to -30°C. The solution was stirred for 30 minutes, then 163 g of the crude acid **3** (1.06 mol) in 100 mL THF was added in 30 minutes at -55 to -30°C, followed by 50 mL THF. The solution was stirred for 15 minutes, the bath was removed and the solution was allowed to warm to -5°C, then it was put in a heating mantle and brought to 50°C in 1 hour. It was kept at 50°C for 2 h, then cooled in an ice-bath. Dry air was bubbled through for 3 days, resulting in an instirrable gel-like solid. The volume had decreased by ca. 50%. Toluene (200 mL) was added, followed by 400 g ice, resulting in a stirrable suspension. Sodium sulfite (152 g, 1.206 mol) was added in portions in 5 minutes and the mixture was stirred for 1.5 h. Toluene (200 mL) was added, the layers were separated, the lower layer being a suspension. The lower layer was extracted with 300 mL toluene and the successive upper layers were washed with 2 x 100 mL water. DCM (500 mL) was then added to the combined aqueous layers, the mixture was cooled in ice and 440 mL 37% hydrochloric acid was added in portions at 15 - 22°C. The layers were separated and the aqueous layer was extracted with 2 x 250 mL DCM. Drying (negative peroxide test), rotary evaporation at 60°C and Kugelrohr at 55°, gave 164.57 g of **4** (0.967 mol, 91%), as a viscous oil. ¹H-NMR (400 MHz, CDCl₃): *δ* 5.83 (dt, *J=* 10.9, 6.6 Hz, 1 H), 5.62 (dt, *J=* 10.7, 8.4 Hz), 2.48 - 2.32 (m, 2 H), 2.31 - 2.09 (m, 3 H), 2.05 - 1.84 (m, 3 H), 1.84 - 1.71 (m, 1 H), 1.69 - 1.45 (m, 1 H).

Advantageously, the direct transformation of **3** to **4** eliminates two reaction steps compared to the method in WO2020256546.

### Enantiomeric resolution of 4 into 4-E^{I} and 4-E^{II}

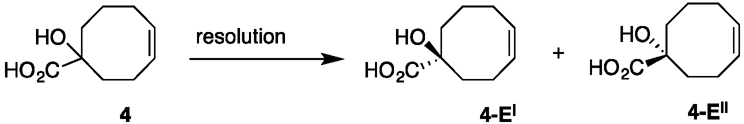

A wide range of resolving agents were screened in different solvents (H₂O, MeCN, H₂O:EtOH 1:1, EtOH, IPA, MEK, iPrOAc, dioxane) on a small scale (0.015 mmol) for the resolution of **4,** but most of them either did not lead to the formation of any crystals or the crystals formed did not lead to substantial enrichment of the enantiomeric purity (table 1). Only in the case of L-valinol and (S)-(-)-diphenyl-2-pyrrolidinemethanol a significant increase in the enantiomeric purity was observed and these two resolving agents were further explored on larger scale (0.5-1 g scale). The screening condition that gave the best combination of degree of enantiomeric resolution and yield after isolation of the solid was resolving agent (S)-(-)-diphenyl-2-pyrrolidinemethanol in tetrahydrofuran (THF). In this experiment, mixing of 897 mg acid **4** (5.27 mmol) and 1.34 g (S)-(-)-diphenyl-2-pyrrolidinemethanol in THF (20 mL) had resulted in fast formation of a solid. After stirring for another 2 hours, the solid had been filtered and the solid had been washed with THF. HPLC analysis had demonstrated a 72/28 enantiomeric ratio for the isolated solid and an 18/82 enantiomeric ratio for remaining **4** present in the filtrate.

**Table 1. Screening conditions for enantiomeric resolution of acid 4.**

| Resolving agent | CAS | HIT |
|---|---|---|
| (*S*)-2-Amino-1-propanol (L-alaninol) | 2749-11-3 | |
| (R)-(+)-1-Phenylethylamine | 3886-69-9 | |
| L-(-)-2-Amino-1-butanol | 5856-63-3 | |
| (1R,2S)-(-)-Ephedrine | 299-42-3 | |
| (S)-(+)-2-Amino-3-methyl-1-butanol (L-Valinol) | 2026-48-4 | + |
| (*S*)-(-)-N-Benzyl-alpha-methylbenzylamine | 17480-69-2 | |
| (+)-Dehydroabietylamine (60%) | 1446-61-3 | |
| (1S,2S)-(+)-2-Amino-1-phenyl-1,3-propanediol | 28143-91-1 | |
| (R)-(+)-3-Pyrrolidinol | 2799-21-5 | |
| (S)-(+)-2-Pyrrolidinemethanol (L-Prolinol) | 23356-96-9 | |
| (S)-(-)-1-(1-Naphthyl)ethylamine | 10420-89-0 | |
| (R)-1-Amino-2-propanol | 2799-16-8 | |
| L-Proline amide | 7531-52-4 | |
| (1R,2R)-(-)-Pseudoephedrine | 321-97-1 | |
| L-Phenylalaninol | 3182-95-4 | |
| (1R,2R)-2-Amino-1-(4-nitrophenyl)propane-1,3-diol | 716-61-0 | |
| Cinchonine | 118-10-5 | |
| Quinidine | 56-54-2 | |
| Quinine | 130-95-0 | |
| Cinchonidine | 485-71-2 | |
| (S)-(+)-L-Phenylglycinol | 20989-17-7 | |
| (R)-(-)-2-Phenylglycine amide | 6485-67-2 | |
| (R)-(+)-2-Phenylpropylamine | 28163-64-6 | |
| L-Phenylalanine amide | 5241-58-7 | |
| (1R,2R)-(-)-1,2-Diaminocyclohexane (0.5 eq) | 20439-47-8 | |
| (-)-Nicotine (1 eq) | 54-11-5 | |
| (1R,2R)-(-)-1,2-Diaminocyclohexane (1 eq) | 20439-47-8 | |
| (1S,2R)-(+)-2-Amino-1,2-diphenylethanol (1 eq) | 23364-44-5 | |
| (S)-(-)-1-Benzyl-3-pyrrolidinol (1 eq) | 101385-90-4 | |
| (S)-1-(4-Bromophenyl)ethylamine (1 eq) | 27298-97-1 | |
| (R)-(-)-1-Cyclohexylethylamine (1 eq) | 5913-13-3 | |
| (R)-(-)-1-Aminoindane (1 eq) | 10277-74-4 | |
| (S)-(-)-alpha,4-Dimethylbenzylamine (1 eq) | 27298-98-2 | |
| (1S, 2*s*-(+)-Thiomicamine (1 eq) | 16854-32-3 | |
| (S) Diphenyl-2-pyrrolidine methanol (1 eq) | 112068-01-6 | + |
| (S)-(-)-1-(2-Naphthyl)ethylamine (1 eq) | 3082-62-0 | |

Subsequently, the method was applied to larger scale: (S)-(-)-diphenyl-2-pyrrolidinemethanol (100.0 g, 0.395 mol) was stirred mechanically with 400 mL THF in a 2L flask to give a cloudy solution. Racemic acid **4** (69.89 g, 0.411 mol) in 350 mL luke-warm THF was added. This rapidly resulted in a suspension. Another 250 mL THF was added and the suspension was stirred for 2.5 h, filtered, and the solid was washed with 200 mL THF. The solid was mixed with 1.7 L THF and the suspension was mechanically stirred for 1.5 h at 60 - 65°C, then stirred for 2 h without heating, and then allowed to stand for 16 h. Filtration and washing with 300 mL THF resulted in 57.8 g solid comprising **4** with an ee of 78%, denoted as **Enantiomer A,** i.e. **4A.** The 57.8 g solid was stirred and heated under reflux for 1 h with 1.5 L THF, then stirred for 2 h at rt, then filtered. The solid (ee 95%) was heated with 1.1 L THF for 45 min at 60 - 65°C, then stirred for 16 h at rt. Filtration and washing with 100 mL THF gave a solid, which was heated with 1.1 L THF for 2 h, then stirred overnight at rt. Filtration and washing with 100 mL THF gave a solid, which was heated once more with 1.1 L THF for 2 h, then stirred for 16 h at rt. Filtration and washing with 100 mL THF gave 29.35 g comprising **4A** in 99 % ee.

### Liberation of the acid 4A from the diphenylprolinol salt:

The 29.35 g (69.3 mmol) salt was stirred with 100 mL toluene and 100 mL 1.5 N sodium hydroxide for 2 h. TBME (50 mL) was added to get a good separation. The layers were separated, and the upper layer was extracted with 2 x 25 mL water. The combined water layers were extracted with 50 mL TBME. TBME (100 mL) was added to the water layer, followed by 20 mL 37% hydrochloric acid. The layers were separated, and the upper layer was washed with 15 mL water. The successive water layers were extracted with 100 mL TBME. Drying and rotary evaporation gave 11.70 g of **4A** as readily solidifying oil (99% ee, 68.7 mmol, 17% overall yield).

In a similar fashion, using 44.68 g recovered acid **4** (0.262 mol; obtained from filtrates of the resolution with (S)-diphenylprolinol, i.e. partially enriched in the other enantiomer) was heated for 90 min with 66.2 g (R)-diphenyl-2-pyrrolidinemethanol (0.261 mol) and 1.5 L THF, then stirred at rt for 2 h. The suspension was filtered and the solid was washed with 100 mL THF. The solid was brought to gentle reflux with 1.5 L THF, then stirred at rt overnight. Filtration and washing with 250 mL THF gave a solid comprising **4B** with 90 % ee. This was heated for 1 h with 1.5 L THF, then stirred overnight at rt. Filtration and washing with 150 mL THF gave a solid, which was heated for 3 h with 1.2 L THF and then stirred at rt overnight. Filtration and washing with THF gave 33.72 g of a solid comprising **4B** with 99 % ee.

### Liberation of the acid 4B from the diphenylprolinol salt:

The 33.72 g salt was stirred for 90 min with 200 mL TBME, 19 mL 30% sodium hydroxide and 80 mL water. A clear mixture was readily formed. The layers were separated and the upper layer was extracted with 2 x 30 mL water. The successive water layers were extracted with 100 mL TBME. The combined aqueous layers were mixed with 150 mL TBME and then acidified with 25 mL 37% hydrochloric acid. The layers were separated and the lower layer was extracted with 100 mL TBME. Drying and rotary evaporation gave 14.78 g of **4B** as a readily solidifying oil (99 % ee, 86.8 mmol, 21%).

### Assignment of absolute configuration of Enantiomer A and Enantiomer B

This assignment of the absolute configuration of Enantiomer A and Enantiomer B can be routinely performed by *e.g.* NMR analysis or crystallization and X-ray diffraction analysis. For the purpose of this example section, it shall be understood that when it is determined that **Enantiomer A (4A)** equals **4-E^{I}** that **Enantiomer B (4B)** then equals **4-E^{II}**, and conversely, when **Enantiomer B** equals **4-E^{I}** that **Enantiomer A** then equals **4-E^{II}**.

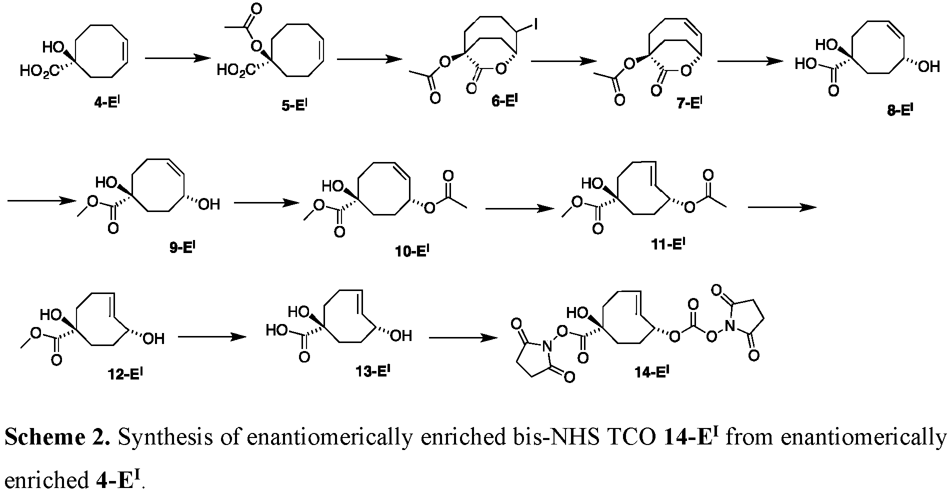

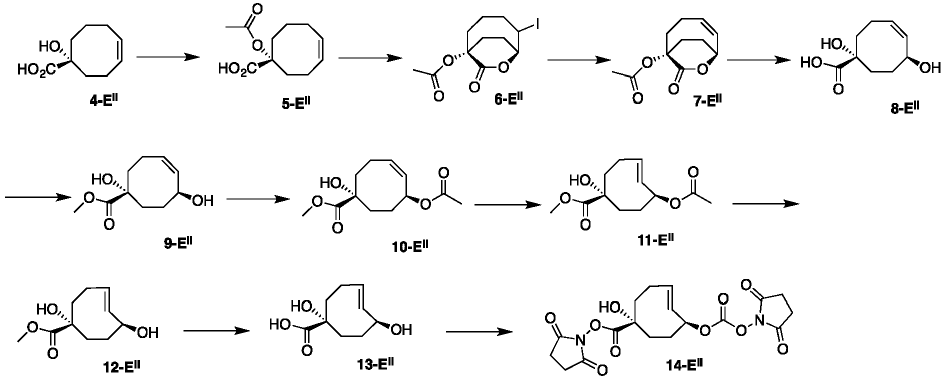

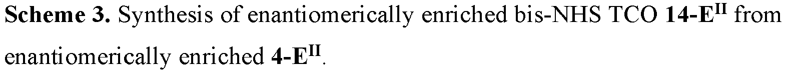

### Compound 5A

**4A** (11.70 g, 68.7 mmol) was stirred with 28.7 g pyridine (362.8 mmol) and 80 mL toluene. DMAP (100 mg) was added, the solution was cooled in ice and acetic anhydride (33.5 g, 0.328 mol) was added dropwise in 30 min. The solution was stirred for 3 d, then put in ice. Toluene (50 mL) and 75 g ice were added, followed by 34 mL 37% hydrochloric acid (in 30 minutes). The mixture was stirred for 30 minutes, the layers were separated and the aqueous layer was extracted with 2 x 75 mL toluene. Drying and rotary evaporation gave 15.79 g of crude **5A.** ¹H-NMR (600 MHz, CDCl₃): *δ* 5.76 (dt, *J =* 10.9, 6.4 Hz, 1 H), 5.55 (q, *J =* 9.1 Hz, 1 H), 2.48 - 2.36 (m, 2 H), 2.34 - 2.21 (m, 2 H), 2.20 - 2.04 (m, 3 H), 2.12 (s, 3H), 2.03 - 1.94 (m, 1 H), 1.59 - 1.50 (m, 2 H).

### Compound 5B

Prepared from **4B** following the protocol for **5A.** ¹H-NMR as for **5A.**

### Compound 5 (racemic)

Prepared from racemic **4** following the protocol for **5A.** ¹H-NMR as for **5A.**

### Compound 7A

The 15.79 g **5A** (max. 68.7 mmol) was dissolved in 150 mL DCM. Water (100 mL) was added, followed by 25 g sodium bicarbonate (0.298 mol) and 50 mL water. The mixture was stirred well for 1 h, then cooled in ice-water. A mixture of iodine and potassium iodide (22.35 resp. 15.31 g; 88.0 resp. 92.2 mmol) was added in 2 equal portions with a 30 min interval and the mixture was stirred overnight. Sodium metabisulfite was added in portions (2.4 g needed to decolorize). The layers were separated, and the upper layer was extracted with 2 x 100 mL DCM. Drying and rotary evaporation gave 24.29 g light-yellow solid iodolactone **6A.**

This was heated with 75 mL toluene and 21.8 g DBU (0.143 mol), first 2 h gradually from 60 to 80°C, then for 9 h at 80°C. The mixture was cooled and poured into 50 g ice-water and 50 mL toluene. The layers were separated, and the lower layer was extracted with 75 mL toluene. The successive organic layers were washed with 50 mL water. Drying and rotary evaporation gave 11.83 g solid olefinic lactone **7A** (56.3 mmol, 82%). ¹H-NMR (400 MHz, CDCl₃): *δ* 5.93 (ddd, *J =* 12.1, 9.2, 5.0 Hz, 1 H), 5.45 (ddd, *J =* 12.0, 4.7, 2.6 Hz, 1 H), 5.16 (s, 1 H), 2.54 - 2.33 (m, 3 H), 2.24 - 2.12 (m, 4 H), 2.10 (s, 3 H), 1.69 - 1.61 (m, 1 H).

The absolute configuration of compound **6A** was determined using single crystal X-ray diffraction (see **Figure 1**). For 6A, the absolute configuration is 1S, 5R, 6R).

### Compound 7B

Prepared from **5B** following the protocol for **7A.** ¹H-NMR as for **7A.**

### Compound 7 (racemic)

Prepared from racemic **5** following the protocol for **7A.** ¹H-NMR as for **7A.**

### Compound 10A

Lactone **7A** (11.83 g, 56.3 mmol) was stirred with 60 mL methanol. Potassium hydroxide (7.82 g, 118.7 mmol) was added and the mixture was stirred for 15 minutes, then heated for 90 min at 60°C. Ethyl acetate (3.5 mL) was added and the solution was heated for 15 minutes, then rotary evaporated at 60°C. DMF (80 mL) was added and the mixture was rotary evaporated, first at 60°, then at 70°C in order to remove traces of water, methanol and some of the DMF. The suspension containing **8A** was cooled in ice and 22 g iodomethane (155.0 mmol) was added in 15 minutes. The mixture was stirred for 2 days, resulting in a clear solution containing **9A.** Pyridine (40 g, 506 mmol) was added, followed by 10 mL toluene, and the solution was stirred for 40 minutes. DMAP (170 mg) was added, the solution was cooled in ice and acetic anhydride (46.3 g, 0.453 mol) was added in 30 minutes, followed by 25 mL toluene. The cooling-bath was removed after 2 h and the solution was stirred for 22 h. It was then poured into a mixture of 200 mL toluene, 50 mL 37% hydrochloric acid and 160 g ice. The mixture was shaken, the layers were separated and the organic layer was washed with 50 mL water. The successive aqueous layers were extracted with 150 mL toluene. Drying and rotary evaporation gave, after heating at 60°C under high vacuum in the Kugelrohr, 12.85 g of **10A** (53.03 mmol, 94%) containing some diacetate analog **15** (see further below). ¹H-NMR (CDCl₃): δ 5.95 (m, 1H), 5.8 (m, 1H), 5.4 (dd, 1H), 3.8 (s, 3H), 3.05 (s, 1H), 2.6 (m, 1H), 2.2 (m, 1H), 2.15 - 1.6 (m) and 2.0 (s) (8H), 1.5 (m, 1H).

### Compound 10B

Prepared from **7B** following the protocol for **10A.** ¹H-NMR as for **10A.**

### Compound 10 (racemic)

Prepared from racemic **7** following the protocol for **10A.** ¹H-NMR as for **10A.**

### Compound 11A

UV irradiation of **10A** (12.85 g, 53.03 mmol), 15.05 g methyl benzoate (110.6 mmol), 500 mg 2,6-di-tert-butyl-4-methylphenol in 750 mL heptane / ethyl acetate, 3 / 1 was done for 33 h, the solution being continuously flushed through a 96 g 10% silver nitrate on silica column (56.5 mmol Ag). The column was eluted with 75 mL TBME and with 275 mL TBME / 10% methanol. The eluate was stirred for 10 minutes with 15 g sodium chloride in 100 mL water, the layers were separated and the water layer was extracted with 100 mL TBME. Drying and rotary evaporation gave 10.7 g of a mixture of **10A** and methyl benzoate. The column material was then stirred with the water layer (diluted with 150 mL water and 5 mL 25% ammonia) and with 200 mL TBME. Filtration, layer separation, drying and rotary evaporation gave 5.0 g residue (the TBME treatment was repeated twice). Chromatography on 80 g silica, starting with 10% ethyl acetate in heptane and gradually increasing the ethyl acetate content, afforded the desired axial isomer of **11A** , followed by diacetate analog **16** (together 2.20 g), followed by the equatorial isomer of **11A.** ¹H-NMR of axial **11A** (CDCl₃): δ 5.9-5.7 (m, 2H), 5.3 (s, 1H), 3.75 (s, 3H), 3.0 (s, 1H), 2.5 (m, 1H), 2.3 - 1.8 (m), 2.1 (s) (8H), 1.7-1.55 (m, 2H); ¹H-NMR of axial diacetate **16A** (CDCl₃): δ 5.9 (m, 1H), 5.5 - 5.6 (dd, 1H), 5.3 (s, 1H), 3.7 (s, 3H), 2.6 (broad d, 1H), 2.45 - 1.6 (m), 2.2 (s) and 2.1 (s) (13H). ¹H-NMR of equatorial **11A** (CDCl₃): δ 5.95 (m, 1H), 5.8 (m, 1H), 5.4 (dd, 1H), 3.8 (s, 3H), 2.7 (m) and 2.6 (s) (2H), 2.2 (m, 1H), 2.15-1.6 (m) and 2.0 (s) (8H), 1.5 (m, 1H).

### Compound 11B

Irradiation of **10B** (15.53 g, 64.1 mmol), 13.6 g methyl benzoate (100 mmol), 600 mg 2,6-di-tert-butyl-4-methylphenol in 750 mL heptane / ethyl acetate 2 / 1 was performed for 82 h, the solution being continuously flushed through a 100 g 10% silver nitrate on silica column (58.8 mmol Ag). Ethyl acetate / heptane 4 / 1 (400 mL) was added to the reactor, flushed through the system, and rotary evaporated. The residue was mainly methyl benzoate (containing ca. 15% **10B**). The column was eluted with 400 mL TBME and the eluate rotary evaporated, affording several grams of residue, mainly **10B.** The column was then eluted with 2 x 250 mL TBME / 10% methanol. The separate eluates were stirred for 10 minutes with 20 g sodium chloride in 100 mL water and the layers were separated. Drying and rotary evaporation gave a mixture of cis and trans isomers as the first fraction, followed by the trans isomers as the second fraction. The column material was then stirred with the water layer (diluted with 150 mL water and 3 mL 25% ammonia) and with 200 mL TBME. Filtration, layer separation, drying and rotary evaporation gave the trans isomers (this TBME treatment was repeated twice). The combined trans fractions were chromatographed on 76 g silica, starting with 8% ethyl acetate in heptane and gradually increasing the ethyl acetate content. The desired axial isomer eluted first, followed by mixed fractions of the axial isomer and the axial diacetate, followed by the equatorial and cis isomer **10B.** The fractions with the axial isomer weighed 3.15 g, the fractions with a mixture of the axial isomer and the axial diacetate weighed 1.93 g. ¹H-NMRs as for **11A.**

### Compound 11 (racemic)

Prepared from racemic **10** following the protocol for **11A.** ¹H-NMR as for **11A.**

### Compound 12A

The axial isomer **11A** was combined with the axial diacetate product **16A** (together 2.20 g) and dissolved in 30 mL methanol. Sodium methoxide (770 mg, 14.26 mmol) was added, the solution was stirred for 90 min and then rotary evaporated at 45°C. Methanol (10 mL) was added to the residue and the solution was evaporated again. TBME (10 mL) was added and the mixture was completely rotary evaporated. TBME (30 mL) was added, followed by 879 mg acetic acid and the mixture was stirred until homogeneous. The suspension was then poured onto a 30 g silica column. Elution was done with TBME, then with 10% methanol in TBME. The first fraction (1.54 g) still contained 15-20% acetate by NMR. This was stirred for 4 h with 9.6 mmol sodium methoxide in 10 mL and then slowly rotary evaporated at 45°C in 30 min. The residue was diluted with 10 mL TBME, 580 mg acetic acid was added (9.67 mmol) and the mixture was stirred for 1 h. The suspension was mixed with 2 g silica, then chromatographed on 20 g silica, using TBME as eluent, then 5% methanol increasing to 10%. This gave 3.06 g of the desired axial isomer **12A.** ¹H-NMR (400 MHz, MeOD): *δ* 5.97 (ddd, *J =* 15.2, 11.2, 3.4 Hz, 1 H), 5.75 (dd, *J =* 16.5, 2.4 Hz, 1 H), 4.41 (dd, *J* = 2.7, 1.3 Hz, 1 H), 3.71 (s, 3 H), 3.37 (s, 1 H), 2.47 (m, 1 H), 2.27 - 2.17 (m, 1 H), 2.15 - 1.91 (m, 4 H), 1.80 - 1.67 (m, 1 H), 1.61 (dd, *J =* 15.3, 6.3 Hz, 1 H).

The equatorial **11A** was dissolved in 10 mL methanol. Sodium methoxide (300 mg) was added and the solution was stirred for 1 h at rt, then slowly rotary evaporated at 40°C in 30 min. TBME was added to the residue, as well as 315 mg acetic acid. The material was then chromatographed on 20 g silica, using TBME as eluent (adding methanol later), affording pure equatorial **12A.** ¹H-NMR (400 MHz, MeOD): *δ* 5.88 (ddd, *J* = 15.8, 11.2, 4.1 Hz, 1 H), 5.43 (dd, *J =* 16.4, 9.3 Hz, 1 H), 4.13 (td, *J =* 9.6, 5.2 Hz, 1 H), 3.78 (s, 3 H), 3.37 (s, 1 H), 2.85 - 2.69 (m, 1 H), 2.38 - 2.28 (m, 1 H), 2.22 - 2.11 (m, 1 H), 2.11 - 2.02 (m, 1 H), 2.01 - 1.84 (m, 2 H), 1.78 - 1.67 (m, 1 H), 1.63 - 1.50 (m, 1 H).

### Compound 12B

The axial isomer **11B** (3.15 g, 3.00 mmol) was dissolved in 25 mL methanol. Sodium methoxide solution was added (3.0 g, 1 mmol/g) and the solution was stirred for 3 h, then rotary evaporated for 1 h at 45°C, with slow removal of solvent. NMR indicated almost complete removal of the acetate, affording **12B.**

The 1.93 g axial isomer **11B** mixed diacetate **16B** was dissolved in 25 mL methanol. Sodium methoxide solution was added (3.0 g, 1 mmol/g) and the solution was stirred for 3 days (NMR indicated that the more hindered acetate was not yet completely removed). To the almost completely deacetylated product of above, 25 ml methanol was added and the solution was stirred at 45°C for 4 h and then rotary evaporated. The residue was mixed with 30 mL TBME and acetic acid (370 mg, 6.17 mmol) was added and the mixture was stirred for 30 min. Silica (5 g) was added, followed by 5 mL methanol and the mixture was stirred for 1 h to give a suspension. It was chromatographed on 45 g silica, elution being done with TBME, TBME / 5% methanol, and TBME / 10% methanol. Drying and evaporation afforded 4.00 g of the axial isomer **12B.** ¹H-NMRs as for **12A.**

### Compound 12 (racemic)

Prepared from racemic **11** following the protocol for **12A.** ¹H-NMR as for **12A.**

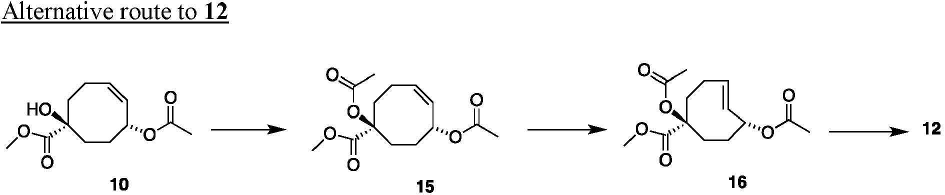

As an alternative to the Z to E isomerization of **10, 10** can be acetylated to diacetate **15** and for Z to E isomerized to afford diacetate **16,** which can then be deprotected to **12.** This allows the use of less polar mobile phase during isomerization and it was discovered that it affords a high axial-equatorial ratio of **16.** Diacetate **15** was obtained by heating 5.1 g monoacetate **10** for 13 h at 100°C with 20 mL acetic anhydride, 5 mL toluene and 55 mg DMAP. Rotary evaporation at 70°C, followed by heating under high vacuum in a Kugelrohr at 70°C and chromatography gave the desired product. ¹H-NMR (CDCl₃): δ 5.85 (m, 2H), 5.45 (dd, 1H), 3.7 (s, 3H), 2.55 (m, 1H), 2.3 - 1.4 (m), 2.2 (s) and 2.0 (s)(13H).

Irradiation of 6.71 g diacetate **15** (23.6 mmol), 9.04 g methyl benzoate (66.4 mmol), 200 mg 2,6-di-tert-butyl-4-methylphenol in 750 mL heptane / ethyl acetate, 3.5 / 1 was done for 44 h, the solution being continuously flushed through a 43.5 g 10% silver nitrate on silica column (25.6 mmol Ag).

The solution was rotary evaporated, the reactor and column being flushed with the heptane / ethyl acetate solvent. Rotary evaporation gave a mixture of methyl benzoate and **15.**

The column was eluted with 100 mL TBME and 150 mL TBME / 5-15% methanol. Each portion was successively stirred with 10 g sodium chloride in 50 mL water and 1 mL 25% ammonia.

Drying and rotary evaporation gave batches of **16** of 0.92 g and 0.48 g, with approximate axial / equatorial ratios of resp. 1/1 4/3. The column material was then stirred with TBME and the water layer (diluted with 100 mL water and 1 mL 25% ammonia). Filtration, layer separation, drying and rotary evaporation gave 2.11 g with an with approximate axial / equatorial ratio of 4/1 (the latter process was repeated twice in order to extract all product). The fractions were combined and dissolved in 30 mL methanol. Sodium methoxide (5 g) in methanol (5 mL) and the solution was stirred for 5 h at 45°C, then stirred at 30°C overnight, and then rotary evaporated at 45°C, to afford solid **12.**

### Compound 13

Lithium hydroxide mono hydrate (115 mg; 2.75 mmol) was dissolved in water (3 mL), and added to a solution of **12 axial** (366 mg; 1.83 mmol) in methanol (5 mL). The mixture was stirred at 20°C in the dark for 18 h. Then it was diluted with water (5 mL), and neutralized by addition of AMBERLITE^{™} IR120 H strong acid cation exchanger (3.66 g). After filtration, the mixture was partly concentrated under reduced pressure to remove methanol. After addition of water (10 mL), the solution was freeze-dried to yield the product **13 axial** as a white powder (316 mg, 93% yield). ¹H-NMR(CD₃OD): δ 5.96 (m, 1H), 5.73 (d, 1H), 4.39 (s, 1H), 2.44 (m, 1H), 2.22 - 1.92 (br.m, 5H), 1.75 - 1.60 (br.m, 2H) ppm. HPLC-MS/PDA (5% to 100% in 10 min): tᵣ=0.86 min (m/z = -185.17 [M-H]⁻ , calcd 186.09 for C₉H₁₄O₄, λₘₐₓ<200 nm).

### Site selective functionalization of Compound 13

The free carboxylic acid of **13 axial** can be selectively reacted with amines, for example to form an amide conjugate to a Construct-B, thus forming a new R₄₇ (with reference to WO2020256546). Subsequently the allylic hydroxyl can for example be bound to a Construct A (e.g. a drug) or a self-immolative linker via carbamate linkage, thus forming a new R₄₈. Exemplary conjugation reactions can be found in Examples of WO2020256546: the preparation of compounds **1.24-1.26.**

### Compound 14

**13 axial** (100 mg; 0.538 mmol) was dissolved in acetonitrile (10 mL), and subsequently DIPEA (346 mg; 2.68 mmol), disuccinimidyl carbonate (690 mg; 2.68 mmol), and DMAP (6.6 mg; 0.054 mmol) were added. The mixture was stirred at 20°C in the dark for 48 h, and the evaporated to dryness and redissolved in chloroform (10 mL). The hazy solution was subsequently washed with 1 M aqueous citric acid (two times 5 mL), and saturated aqueous sodium bicarbonate (two times 5 mL). After drying of the solution over sodium sulfate and filtration, it was evaporated to dryness *in vacuo* to give the axial product as a white solid (150 mg, 66% yield). ¹H-NMR(CDCl₃): δ 6.02 (m, 1H), 5.78 (d, 1H), 5.34 (s, 1H), 2.85 (s, 8H), 2.56 (m, 1H), 2.40 - 2.03 (br.m, 7H) ppm. HPLC-MS/PDA (5% to 100% in 10 min): tᵣ=3.76 min (m/z=+446.92 [M+Na]⁺; calcd 424.11 for C₁₈H₂₀N₂O₁₀; λₘₐₓ<200 nm).

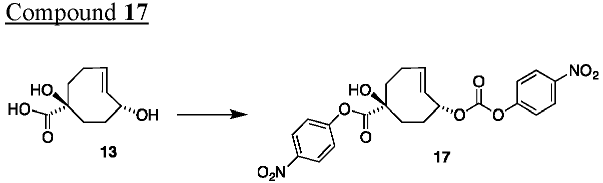

As an alternative to **14,** bis PNP TCO **17** potentially allows for better regioselectivity in the TCO functionalization. TCO **13 axial** in dry MeCN is treated with 4-nitrophenyl chloroformate (6 eq.) and 4-(dimethylamino)pyridine (3eq) at room temperature for 16 hours followed by concentration in vacuo. The residue is chromatographed on silica with heptanes as eluent with increasing amounts of EtOAc. The product fractions of **17** are combined and dried in vacuo.

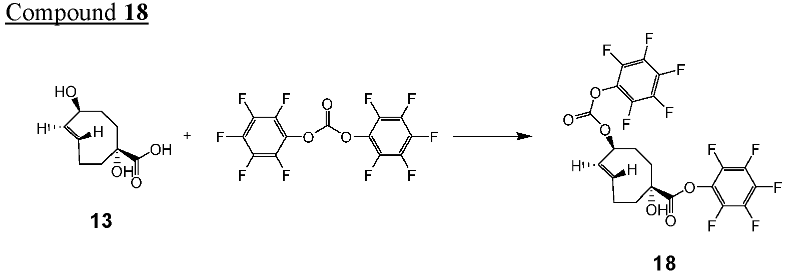

Compound **13 axial** (100 mg; 0.538 mmol) was dissolved in acetonitrile (10 mL), and subsequently DIPEA (346 mg; 2.68 mmol), bis(pentafluorophenyl) carbonate (1.0 g; 2.54 mmol), and DMAP (6.5 mg; 0.054 mmol) were added. The mixture was stirred at 20°C in the dark for 48 h, and evaporated to dryness and redissolved in chloroform (10 mL). The hazy solution was subsequently washed with 1 M aqueous citric acid (two times 5 mL), and saturated aqueous sodium bicarbonate (two times 5 mL). After drying of the solution over sodium sulfate and filtration, it was evaporated to dryness *in vacuo* to give **18** as a colorless oil (182 mg, 60% yield). ¹H-NMR(CDCl₃): δ 6.20 - 5.96 (m, 1H), 5.85 (m, 1H), 5.39 (s, 1H), 2.85 (s, 1H), 2.69 - 2.59 (m, 1H), 2.47 - 2.13 (m, 6H), 2.07 (ddd, 1H) ppm. ¹⁹F NMR (377 MHz, CDCl₃) δ -153.30 (m, 4H), -156.84 (m, 1H), -157.62 (m, 1H), -161.64 (m, 2H), -162.11 (m, 2H) ppm.

### Example 3. Separating the axial from the equatorial isomer after Z-E isomerization (not claimed)

As disclosed in WO2020256546, the axial and equatorial isomers can be separated by silica column chromatography of racemic **12.** In the foregoing example we demonstrated that the axial and equatorial isomers can be separated by silica column chromatography of **11.** Therefore the following racemic and enantiomerically enriched TCOs can be separated into the following axial and equatorial isomers.

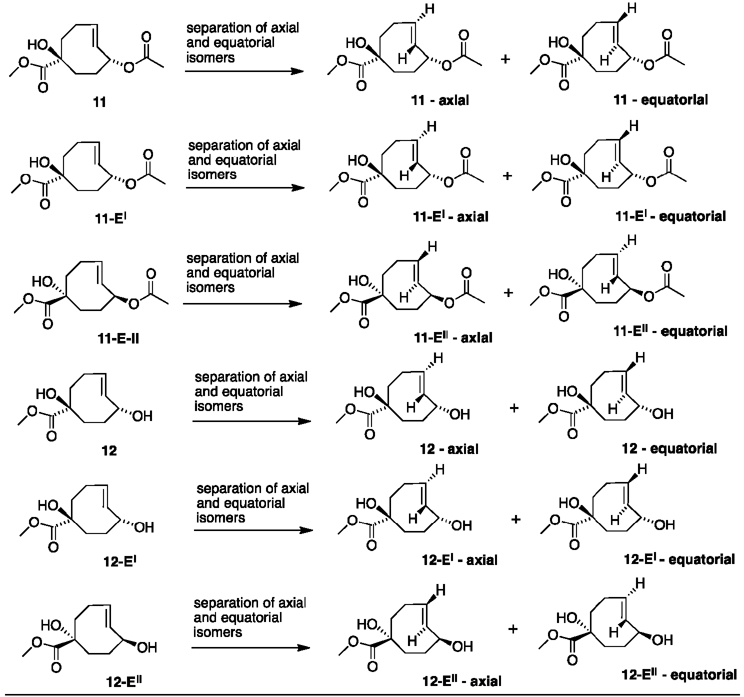

### Example 4. Evaluation of regioselectivity of functionalization of Compound 14 and 18

Preliminary experiments with **14 axial** indicated that the reactivity difference between the NHS carbonate and the NHS ester is much lower than for the analogous trans-cyclooctene with a methyl substituent (Rossin et al., Bioconjugate Chem 2016, 27, 1697-1706; compound 22A) in place of the tertiary hydroxyl. An amine can be selectively reacted with the NHS carbonate of compound 22A and not with the NHS ester, due to the high reactivity difference between the two sites, allowing regioselective control over TCO functionalization. The reduced reactivity difference between the NHS moieties on **14** hampers regioselective functionalization of **14** (i.e. first reaction on the NHS carbonate, then on the NHS ester). To find a solution the following model studies were conducted.

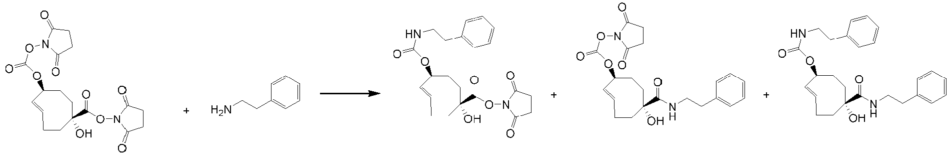

**14 axial** (0.55 mg; 1.3 µmol) was dissolved in acetonitrile (200 µL) at room temperature, and a stoichiometric amount of 2-phenethylamine (43 µL of a 30 mM solution in acetonitrile; 1.3 µmol) was added. The mixture was homogenized, and after 30 min analyzed by HPLC-MS/PDA (10 µL in 1 mL 30% acetonitrile/water) to reveal the formation of a mixture of mono- and twice-reacted products, indeed demonstrating the absence of selectivity with primary amines in these conditions. HPLC-MS/PDA (5% to 100% in 10 min): mono-reacted product: tᵣ=4.42 min (m/z=+431.11 [M+H]⁺; calcd 430.17 for C₂₂H₂₆N₂O₇; λₘₐₓ=256 nm); twice-reacted product: tᵣ=4.73 min (m/z=+437.17 [M+H]⁺; calcd 436.24 for C₂₆H₃₂N₂O₄; λₘₐₓ=256 nm).

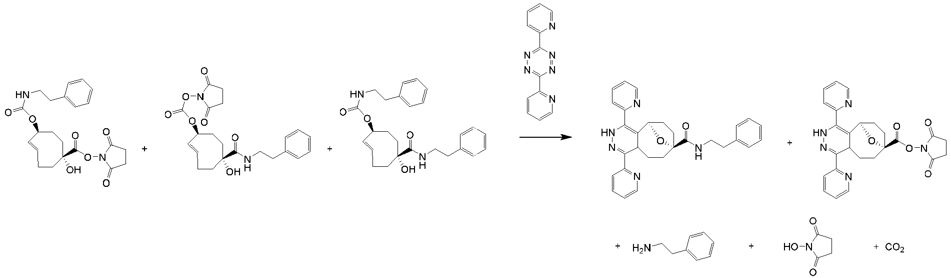

To the previous HPLC-MS/PDA sample, containing a mixture of mono- and twice-reacted products, was added a solution of 3,6-bis(2-pyridinyl)-1,2,4,5-tetrazine (2 µL 25 mM in DMSO) at room temperature. The sample was homogenized and after 5 min analyzed by HPLC-MS/PDA, which proved the formation of two elimination products, containing either the 2-phenethylamide or the N-hydroxysuccinimide ester moiety. HPLC-MS/PDA (5% to 100% in 10 min): N-hydroxysuccinimide ester functionalized elimination product: tᵣ=3.18 min (m/z=+474.25 [M+H]⁺; calcd 473.17 for C₂₅H₂₃N₅O₅; λₘₐₓ=426 nm); 2-phenethylamide functionalized elimination product: tᵣ=3.53 min (m/z=+480.25 [M+H]⁺; calcd 479.23 for C₂₉H₂₉N₅O₂; λₘₐₓ=428 nm).

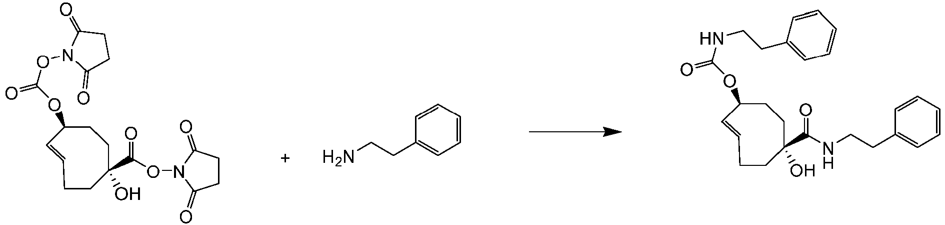

**14** axial (0.65 mg; 1.5 µmol) was dissolved in acetonitrile (100 µL), and an excess of 2-phenethylamine (150 µL of a 30 mM solution in acetonitrile; 4.5 µmol) was added at room temperature. The mixture was homogenized, and after 5 min analyzed by HPLC-MS/PDA (10 µL in 1 mL 30% acetonitrile/water) to reveal the exclusive formation of the twice-reacted compound. HPLC-MS/PDA (5% to 100% in 10 min): tᵣ=4.73 min (m/z=+437.17 [M+H]⁺; calcd 436.24 for C₂₆H₃₂N₂O₄; λₘₐₓ=256 nm).

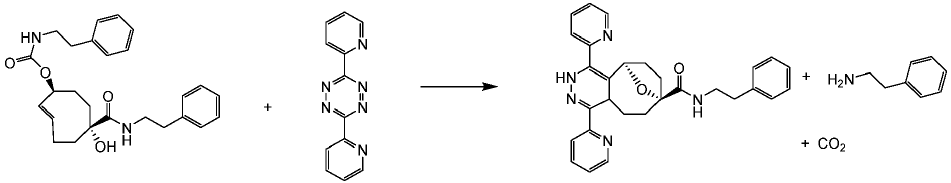

To the previous HPLC-MS/PDA sample was added a solution of 3,6-bis(2-pyridinyl)-1,2,4,5-tetrazine (2 µL 25 mM in DMSO) at room temperature. The sample was homogenized and after 5 min analyzed by HPLC-MS/PDA, which proved the formation of the 2-phenethyl functionalized elimination product. HPLC-MS/PDA (5% to 100% in 10 min): tᵣ=3.55 min (m/z=+480.33 [M+H]⁺; calcd 479.23 for C₂₉H₂₉N₅O₂; λₘₐₓ=429 nm).

The foregoing confirms that there is no or much less regioselectivity in 14 when reacting with primary amines compared to its methyl analog.

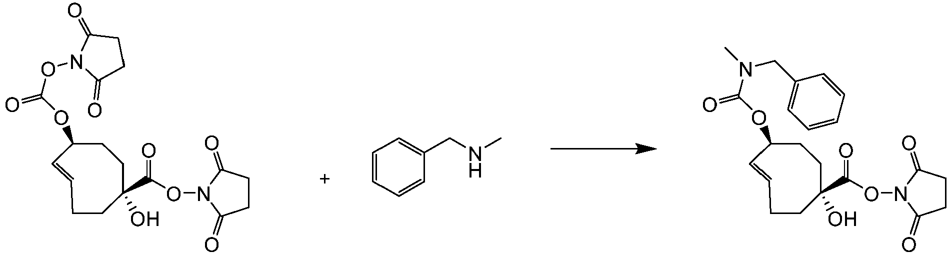

**14 axial** (2.5 mg; 6.0 µmol) was dissolved in acetonitrile (1300 µL) and cooled to -10°C. A stoichiometric amount of N-methylbenzylamine (200 µL of a 30 mM solution in acetonitrile; 6.0 µmol) was added, and the mixture was homogenized, and after 30 min analyzed by HPLC-MS/PDA (100 µL in 0.9 mL 30% acetonitrile/water) to reveal the exclusive formation of the mono-reacted product, demonstrating the high selectivity with secondary amines in these conditions. HPLC-MS/PDA (5% to 100% in 10 min): mono-reacted product: tᵣ=4.38 min (m/z=+453.08 [M+Na]⁺; calcd 430.17 for C₂₂H₂₆N₂O₇; λₘₐₓ=256 nm). This reaction was also regioselectively performed at room temperature, with the same results.

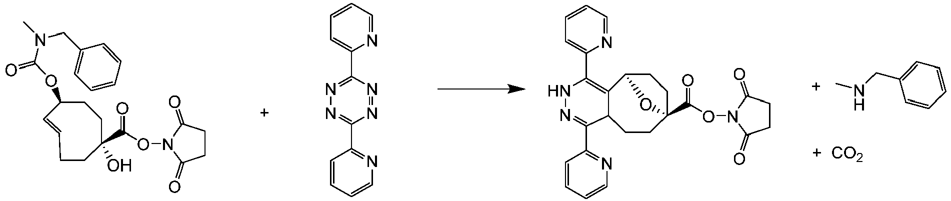

To the previous HPLC-MS/PDA sample, containing the mono-reacted product, was added a solution of 3,6-bis(2-pyridinyl)-1,2,4,5-tetrazine (20 µL 25 mM in DMSO) at room temperature. The sample was homogenized and after 5 min analyzed by HPLC-MS/PDA, which proved the exclusive formation of the elimination product containing the N-hydroxysuccinimide ester moiety. HPLC-MS/PDA (5% to 100% in 10 min): tᵣ=3.17 min (m/z=+474.25 [M+H]⁺; calcd 473.17 for C₂₅H₂₃N₅O₅; λₘₐₓ=425 nm). This demonstrates that the amine had reacted exclusively with the NHS carbonate and not with the NHS ester.

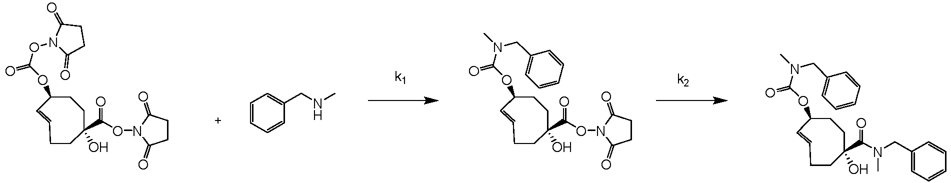

**14 axial** (0.65 mg; 1.5 µmol) was dissolved in acetonitrile (10 µL) at room temperature. An excess of N-methylbenzylamine (150 µL of a 30 mM solution in acetonitrile; 4.5 µmol) was added, and the mixture was homogenized. The progress of the reaction was analyzed by HPLC-MS/PDA (10 µL in 90 µL 30% acetonitrile/water) at different time points, both before and after activation with 3,6-bis(2-pyridinyl)-1,2,4,5-tetrazine (2 µL 25 mM in DMSO). This demonstrated the much higher reactivity of the activated allylic alcohol with respect to the active ester towards secondary amine. The former has reacted completely after 5 min, while the latter has a half life of 300 min.

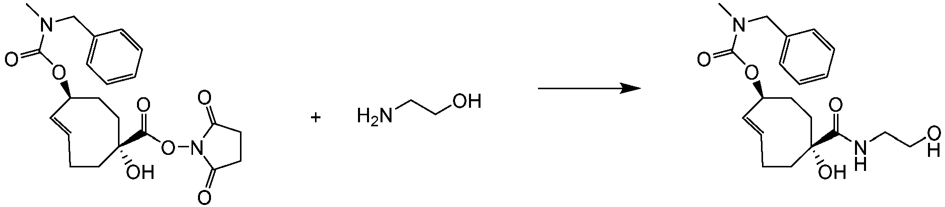

To the reaction mixture containing the mono-reacted secondary amine construct (200 µL 4 mM; 0.80 µmol) was added a solution of 2-aminoethanol (50 µL 48 mM in acetonitrile; 2.4 µmol) at room temperature. The reaction mixture was homogenized, and after 5 min analyzed by HPLC-MS/PDA (10 µL in 90 µL 30% acetonitrile/water) to reveal the exclusive formation of the desired compound. HPLC-MS/PDA (5% to 100% in 10 min): tᵣ=3.71 min (m/z=+377.08 [M+H]⁺, -375.17 [M-H]⁻; calcd 376.20 for C₂₀H₂₈N₂O₅; λₘₐₓ=255 nm).

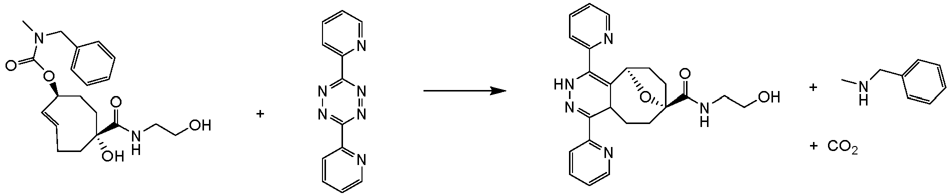

To the previous HPLC-MS/PDA sample was added a solution of 3,6-bis(2-pyridinyl)-1,2,4,5-tetrazine (2.5 µL 25 mM in DMSO) at room temperature. The sample was homogenized and after 5 min analyzed by HPLC-MS/PDA, which proved the exclusive formation of the elimination product containing the 2-aminoethanol moiety. HPLC-MS/PDA (5% to 100% in 10 min): tᵣ=2.63 min (m/z=+420.25 [M+H]⁺; calcd 419.20 for C₂₃H₂₅N₅O₃; λₘₐₓ=426 nm).

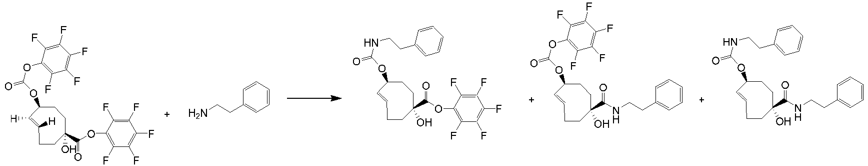

**18 axial** (0.28 mg; 0.5 µmol) was dissolved in acetonitrile (20 µL), and a stoichiometric amount of 2-phenethylamine (20 µL of a 25 mM solution in acetonitrile; 0.5 µmol) was added at room temperature. The mixture was homogenized, and after 30 min diluted with acetonitrile (160 µL) and water (800 µL). The sample was analyzed by HPLC-MS/PDA to reveal the formation of a mixture of mono- and twice-reacted products, demonstrating the absence of selectivity with primary amines at these conditions. HPLC-MS/PDA (5% to 100% in 10 min): mono-reacted product: tᵣ=5.32 min (m/z=+500.17 [M+H]⁺; calcd 499.14 for C₂₄H₂₂F₅NO₅; λₘₐₓ=257 nm); twice-reacted product: tᵣ=4.69 min (m/z=+437.25 [M+H]⁺; calcd 436.24 for C₂₆H₃₂N₂O₄; λₘₐₓ=257 nm).

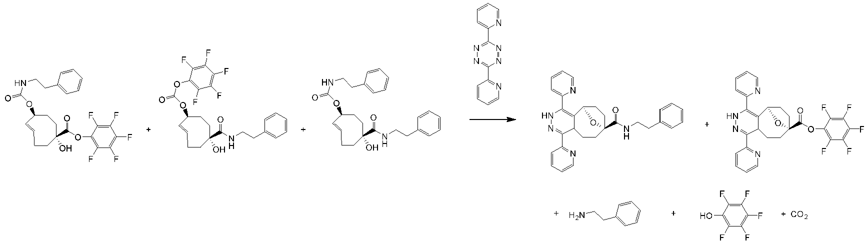

To the previous HPLC-MS/PDA sample, containing a mixture of mono- and twice-reacted products, was added a solution of 3,6-bis(2-pyridinyl)-1,2,4,5-tetrazine (30 µL 25 mM in DMSO, 0.75 µmol) at room temperature. The sample was homogenized and after 5 min analyzed by HPLC-MS/PDA, which proved the formation of two elimination products, containing either the 2-phenethylamide or the pentafluorophenyl ester moiety. HPLC-MS/PDA (5% to 100% in 10 min): pentafluorophenyl ester functionalized elimination product: tᵣ=4.43 min (m/z=+543.33 [M+H]⁺; calcd 542.14 for C₂₇H₁₉F₅N₄O₃; λₘₐₓ=427 nm); 2-phenethylamide functionalized elimination product: tᵣ=3.54 min (m/z=+480.50 [M+H]⁺; calcd 479.23 for C₂₉H₂₉N₅O₂; λₘₐₓ=428 nm).

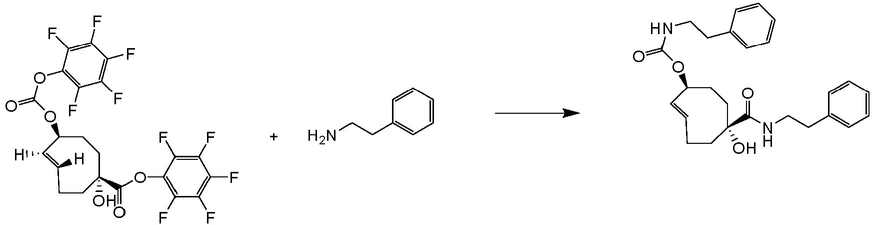

**18 axial** (0.28 mg; 0.5 µmol) was dissolved in acetonitrile (20 µL), and an excess of 2-phenethylamine (60 µL of a 25 mM solution in acetonitrile; 1.5 µmol) was added at room temperature. The mixture was homogenized, and after 15 min diluted with acetonitrile (120 µL) and water (800 µL). The sample was analyzed by HPLC-MS/PDA to reveal the exclusive formation of the twice-reacted compound. HPLC-MS/PDA (5% to 100% in 10 min): tᵣ=4.69 min (m/z=+437.25 [M+H]⁺; calcd 436.24 for C₂₆H₃₂N₂O₄; λₘₐₓ=257 nm).

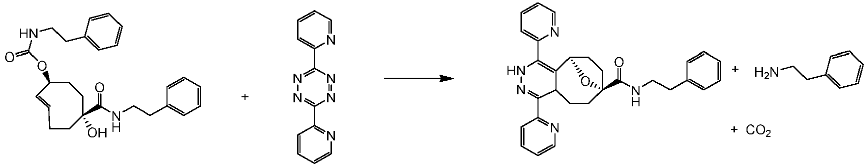

To the previous HPLC-MS/PDA sample was added a solution of 3,6-bis(2-pyridinyl)-1,2,4,5-tetrazine (30 µL 25 mM in DMSO, 0.75 µmol) at room temperature. The sample was homogenized and after 5 min analyzed by HPLC-MS/PDA, which proved the formation of the 2-phenethyl functionalized elimination product. HPLC-MS/PDA (5% to 100% in 10 min): tᵣ=3.54 min (m/z=+480.50 [M+H]⁺; calcd 479.23 for C₂₉H₂₉N₅O₂; λₘₐₓ=428 nm).

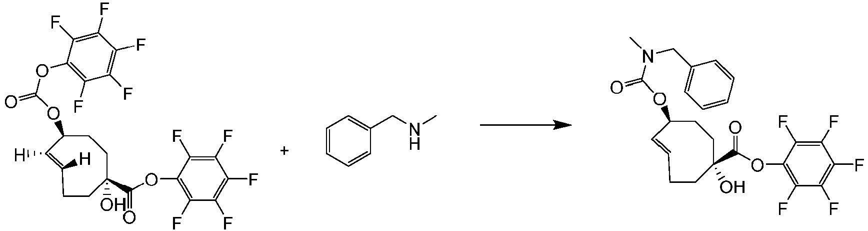

**18 axial** (0.14 mg; 0.25 µmol) was dissolved in acetonitrile (190 µL), and a stoichiometric amount of N-methylbenzylamine (10 µL of a 25 mM solution in acetonitrile; 0.25 µmol) was added at room temperature. The mixture was homogenized, and after 18 hours diluted with water (800 µL). The sample was analyzed by HPLC-MS/PDA to reveal the exclusive formation of the mono-reacted product, demonstrating the high selectivity with secondary amines at these conditions. HPLC-MS/PDA (5% to 100% in 10 min): mono-reacted product: tᵣ=5.46 min (m/z=+500.25 [M+H]⁺; calcd 499.14 for C₂₄H₂₂F₅NO₅; λₘₐₓ=257 nm).

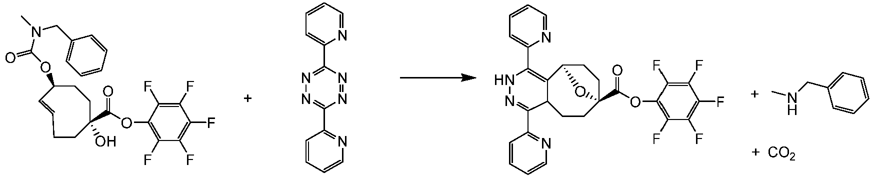

To the previous HPLC-MS/PDA sample, containing the mono-reacted product, was added a solution of 3,6-bis(2-pyridinyl)-1,2,4,5-tetrazine (15 µL 25 mM in DMSO, 0.375 µmol) at room temperature. The sample was homogenized and after 5 min analyzed by HPLC-MS/PDA, which proved the formation of which proved the exclusive formation of the elimination product containing the pentafluorophenyl ester moiety. HPLC-MS/PDA (5% to 100% in 10 min): pentafluorophenyl ester functionalized elimination product: tᵣ=4.43 min (m/z=+543.33 [M+H]⁺; calcd 542.14 for C₂₇H₁₉F₅N₄O₃; λₘₐₓ=428 nm).

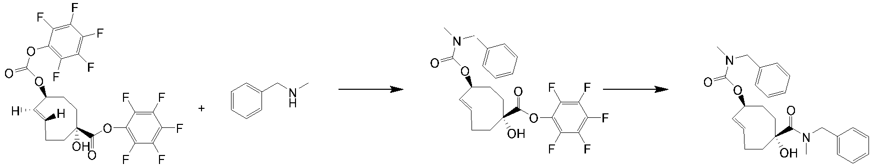

**18 axial** 0.14 mg; 0.25 µmol) was dissolved in acetonitrile (170 µL), and a small excess of N-methylbenzylamine (30 µL of a 25 mM solution in acetonitrile; 0.75 µmol) was added at room temperature. The mixture was homogenized, and after 18 hours diluted with water (800 µL). The sample was analyzed by HPLC-MS/PDA to reveal the formation of a mixture of mono-(65%) and twice-reacted products (35%). HPLC-MS/PDA (5% to 100% in 10 min): mono-reacted product: tᵣ=5.45 min (m/z=+500.25 [M+H]⁺; calcd 499.14 for C₂₄H₂₂F₅NO₅; λₘₐₓ=257 nm); twice-reacted product: tᵣ=5.25 min (m/z=+437.33 [M+H]⁺; calcd 436.24 for C₂₆H₃₂N₂O₄; λₘₐₓ=257 nm).

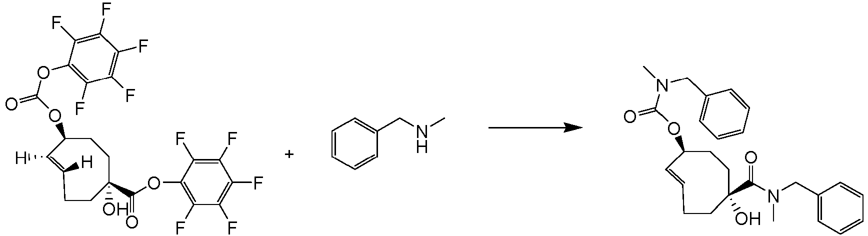

**18 axial** (0.14 mg; 0.25 µmol) was dissolved in acetonitrile (10 µL), and a large excess of N-methylbenzylamine (1.2 mg; 10 µmol) was added at room temperature. The mixture was homogenized, and after 45 min diluted with acetonitrile (160 µL) and water (800 µL). The sample was analyzed by HPLC-MS/PDA to reveal the exclusive formation of the twice-reacted product. HPLC-MS/PDA (5% to 100% in 10 min): tᵣ=5.25 min (m/z=+437.33 [M+H]⁺; calcd 436.24 for C₂₆H₃₂N₂O₄; λₘₐₓ=257 nm).

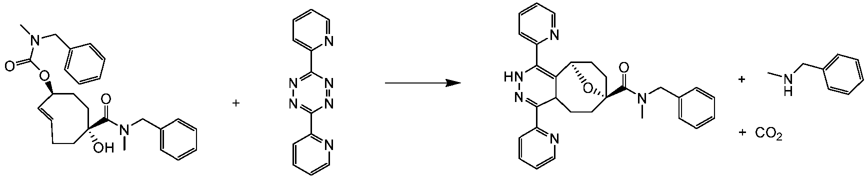

To the previous HPLC-MS/PDA sample was added a solution of 3,6-bis(2-pyridinyl)-1,2,4,5-tetrazine (15 µL 25 mM in DMSO, 0.375 µmol). The sample was homogenized and after 5 min analyzed by HPLC-MS/PDA, which proved the formation of the N-methylbenzyl functionalized elimination product. HPLC-MS/PDA (5% to 100% in 10 min): tᵣ=3.67 min (m/z=+480.42 [M+H]⁺; calcd 479.23 for C₂₉H₂₉N₅O₂; λₘₐₓ=430 nm).

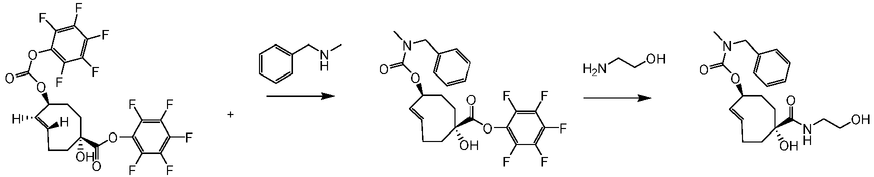

**18 axial** (1.87 mg; 3.33 µmol) was dissolved in acetonitrile (533 µL), and N-methylbenzylamine (2.0 mg; 16.7 µmol) was added at room temperature. The mixture was homogenized and stirred for 10 min at 20°C. Subsequently, 2-aminoethanol (2.0 mg; 33.3 µmol) was added and the mixture was homogenized and stirred for 30 min at 20°C. Analysis by HPLC-MS/PDA (40 µL in 960 µL 20% acetonitrile/water) revealed the exclusive formation of the desired compound. HPLC-MS/PDA (5% to 100% in 10 min): tᵣ=3.73 min (m/z=+377.17 [M+H]⁺, -375.33 [M-H]⁻; calcd 376.20 for C₂₀H₂₈N₂O₅; λₘₐₓ=257 nm).

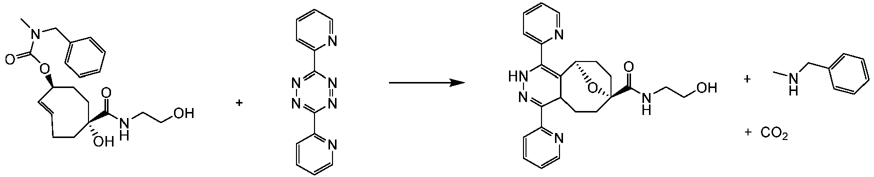

To the previous HPLC-MS/PDA sample was added a solution of 3,6-bis(2-pyridinyl)-1,2,4,5-tetrazine (15 µL 25 mM in DMSO) at room temperature. The sample was homogenized and after 5 min analyzed by HPLC-MS/PDA, which proved the exclusive formation of the elimination product containing the 2-aminoethanol moiety. HPLC-MS/PDA (5% to 100% in 10 min): tᵣ=2.62 min (m/z=+420.33 [M+H]⁺; calcd 419.20 for C₂₃H₂₅N₅O₃; λₘₐₓ=427 nm).

The foregoing demonstrates that **14** and **18** can be regioselectively modified by by increasing steric hindrance through the use of a secondary amine in the reaction with the NHS or PFP carbonate. Intermediates **14** and **18** are therefore suitable for functionalization on the allylic position with for example a secondary amine containing Construct-A or a self-immolative linker, forming a new R48. The NHS or PFP ester position can subsequently be reacted with for example an amine-containing Construct B, forming a new R47. Example 5 demonstrates the use of this finding in the regiospecific conjugation of MMAE (Construct-A, i.e. a Drug) to the allylic TCO position via a releasable carbamate link, followed by reaction of the NHS or PFP ester with a building block for the spacer towards the targeting protein (Construct-B, i.e. a Targeting Agent).

### Example 5. Synthesis of ADC linkers

### Mal-TCO-MMAE-PEG24 (22-PEG24)

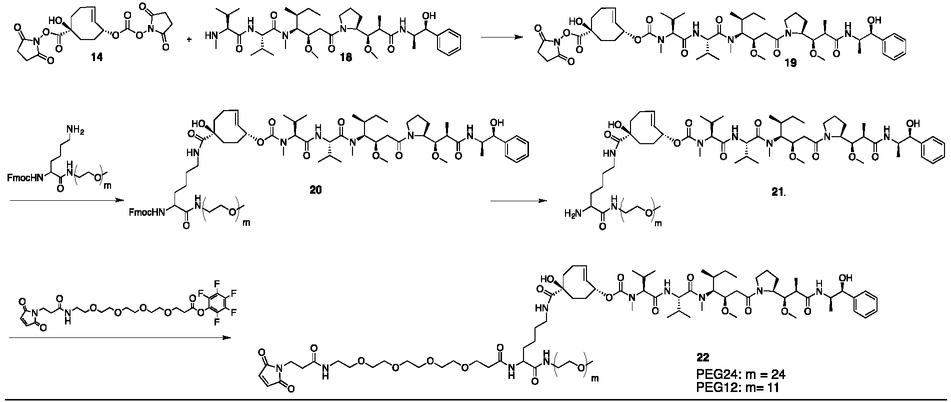

Compound **22-PEG24** was prepared in several steps in situ. To a solution of monomethyl auristatin E (MMAE; 112 mg as mono TFA salt, 135 µmol, Levena Biopharma, **18**) in 2 mL of anhydrous DMF in a glass vial was added bis-NHS-TCO **14 axial** (57 mg, 135 µmol) and DIEA (70 µL, 405 µmol). The mixture was stirred at rt in the dark for 6h, at which point HPLC-MS analysis indicated >90% conversion to intermediate 19. To this reaction mixture was added Fmoc-Lys-PEG₂₄ (as mono TFA salt, 251 mg, 162 µmol, Levena Biopharma) and DIEA (28 µL, 161 µmol). The reaction mixture was stirred at room temperature in the dark for 3 days, at which point HPLC-MS analysis showed formation of compound **20-PEG24** and that all **19** was consumed. To this mixture was added piperidine (150 µL, 1.52 mmol) and the stirring was continued for 20 min, at which point HPLC-MS analysis indicated complete formation of compound **21-PEG24.** The product was purified by preparative HPLC (20 min run, 10 to 70% acetonitrile at 50 mL/min). The collected fractions were analysed by HPLC-MS. The pure fractions were lyophilized in the dark to give **21-PEG24** as acetate salt as a viscous colourless gum (156 mg; 52%). HPLC-MS: *m*/*z* 1064.7 Da [M+2H]²⁺

To a solution of **21-PEG24** (mono acetate salt, 120 mg, 55 µmol) in 3 mL of anhydrous DMF in a glass vial was added Mal-NH-PEG₄-CH₂CH₂COOPFP (32 mg, 55 µmol, Levena Biopharma) and DIEA (19 µL, 109 µmol). The mixture was stirred at room temperature for 10 min in the dark, at which point HPLC-MS analysis indicated complete conversion to compound **22-PEG24.** The reaction was purified by preparative HPLC (20 min run, from 10 to 80% acetonitrile at 50 mL min⁻¹). The collected fractions were analysed by HPLC-MS and the pure fractions were lyophilized in a dark to give **22-PEG24** as viscous gum (61 mg; 45%). HPLC-MS: *m*/*z* 1263.8 Da [M+2H]²⁺

### Mal-TCO-MMAE-PEG12 (22-PEG12)

Prepared as for **22-PEG24,** using Fmoc-Lys-PEG₁₂ (Levena Biopharma). HPLC-MS: *m*/*z* 999.6 Da [M+2H]²⁺

### Mal-PEG24-TCO-MMAE (24-PEG24)

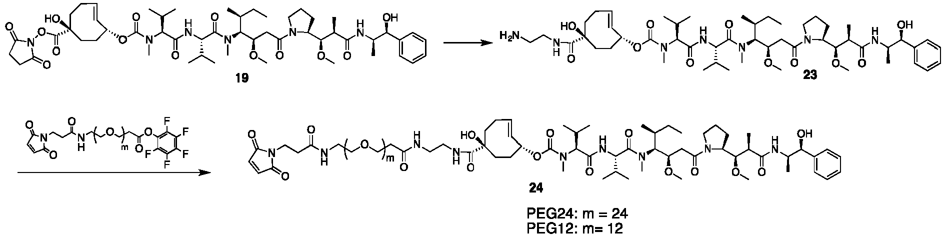

**19** (120 µmol) and ethylenediamine (1.2 mmol) in 2 mL DMF were stirred for 2 h at rt. The product was purified by preparative HPLC (20 min run, 10 to 70% acetonitrile at 50 mL/min). The collected fractions were analysed by HPLC-MS. The pure fractions were lyophilized in the dark to give **23** as a solid (85 µmol). HPLC-MS: *m*/*z* 972.6 Da [M+H]⁺

To a stirred solution of Mal-PEG₂₄-NHS ester (70 mg, 50 µmol, Quanta Biodesign) and **23** (39 mg, 40 µmol) in anhydrous DMF (1 mL) was added DIEA (9 µL). The reaction mixture was stirred at room temperature for 1 h and purified directly by preparative HPLC (20 min run, from 20 to 70% acetonitrile at 50 mL min⁻¹) to give the desired product **24-PEG24** as a colourless syrup after lyophilization (67 mg; 75%). HPLC-MS: *m*/*z* 1126.2 [M+2H]²⁺

### Mal-PEG12-TCO-MMAE (24-PEG12)

Prepared as for **24-PEG24,** using Mal-PEG₁₂-NHS ester (Quanta Biodesign). HPLC-MS: m/z 862.0 Da [M+2H]²⁺

### Example 6. ADC preparation and evaluation

The anti-TAG72 diabody AVP04-58 (MW 51088 Da) was provided by Avipep Ltd. AVP04-58 was prepared as previously described (Li et al., Bioconjug. Chem. 2011, 22, 709). without the C-terminal His₆ tag, and differs from the parent anti-TAG72 diabody AVP04-07 (Li et al., Bioconjug. Chem. 2011, 22, 709) by the substitution of cysteine residues into positions L8 and L11 in the VL domain, thus affording two additional cysteine residues per scFv monomer (4 cysteines per diabody). These 4 cysteine residues were used for site-specific conjugation of maleimide-TCO-MMAE linker drugs **22/24,** as described below.

| **Diabody** | **Diabody sequence (SEQ ID NO:1)** |
|---|---|
| TAG72-binding diabody derived from the CC49 antibody | SVQLQQSDAELVKPGASVKISCKASGYTFTDHA IHWVKQNPEQGLEWIGYFSPGNDDFKYNERFK GKATLTADKSSSTAYLQLNSLTSEDSAVYFCTR SLNMAYWGQGTSVTVSSGGGGSDIVMTQSCSS CPVSVGEKVTLSCKSSQSLLYSGNQKNYLAWY QQKPGQSPKLLIYWASTRESGVPDRFTGSGSGT DFTLSISSVETEDLAVYYCQQYYSYPLTFGAGTK LVLKR |

A ca. 2 mg/mL solution of the anti-TAG72 diabody AVP0458 in degassed 100 mM phosphate buffer pH 6.8 containing 2 mM EDTA (EDTA-PBS) was combined with freshly dissolved 100 mM DTT in EDTA-PBS (6 mM final DTT concentration) and incubated at room temperature for 1 h. The reduced diabody was then loaded on a PD-10 column and eluted from the column with EDTA-PBS, and immediately afterwards added with a solution of linker-drug **22/24** (ca. 7.5 equiv. per SH) in DMSO (ca. 15% DMSO v/v% in the final mixture) and incubated overnight at 4°C. Subsequently the ADCs were purified by gel filtration on a AKTA system equipped with a Superdex 75 26/60 prep-grade column which was eluted with EDTA-PBS at 2.5 mL/min. The purified fractions were combined and mixed with 5% DMSO. The solution was then concentrated using Amicon Ultra-15 centrifugal filters and the ADC concentration was measured by NanoDrop. ESI-TOF MS confirmed the identities of the conjugates with DARs of 4 in the presence of only trace amounts of aggregates. AVP0458-**22-PEG24:** monomer mass 30600 Da; AVP0458-**22-PEG12:** monomer mass 29543 Da; AVP0458-**24-PEG24:** monomer mass 30050; AVP0458-**24-PEG12:** monomer mass 28992 Da.

ADC stock solutions (10 µL 2 µg/µl in 5% DMSO/EDTA-PB) were diluted with PBS (90 µL), mixed with tetrazine **2.4** from WO2020256546 (5 µL 2.5 mM in PBS) and incubated at 37°C for 1 h. Subsequent HPLC-QTOF-MS analysis demonstrated the formation of free MMAE (*m*/*z*=+718.51 Da) and the diabody reaction products without MMAE, indicating release yields of >92%.

### Example 7. Maleimide-TCO-MMAE linker drugs and corresponding diabody conjugates

The following schemes depict examples of the racemic and enantiomerically enriched maleimide-TCO-MMAE linker drugs and corresponding AVP0458 conjugates that can be prepared with the presented method. Herein, "racemic and enantiomerically enriched" refers to the two chiral centers directly on the cyclooctene ring, and the chiral center on the alpha carbon on the lysine residue in the maleimide-PEG linker. Thus, it will be understood that "racemic and enantiomerically enriched" does not refer to the chiral centers in the MMAE residue.

Herein n is a value higher than 0 and at most 4, preferably a value ranging from 2 to 4, more preferably ranging from 3 to 4, even more preferably ranging from 3.5 to 4, most preferably n is 4. Herein, it is understood that if n > 1, the AVP0458 is coupled to multiple TCO-MMAE moieties. It is not meant that if n > 1, multiple TCO-MMAE moieties form a linear polymer.

Also disclosed, but not part of the claimed invention are the structures presented below, and pharmaceutically acceptable salts, tautomers, and solvates thereof.

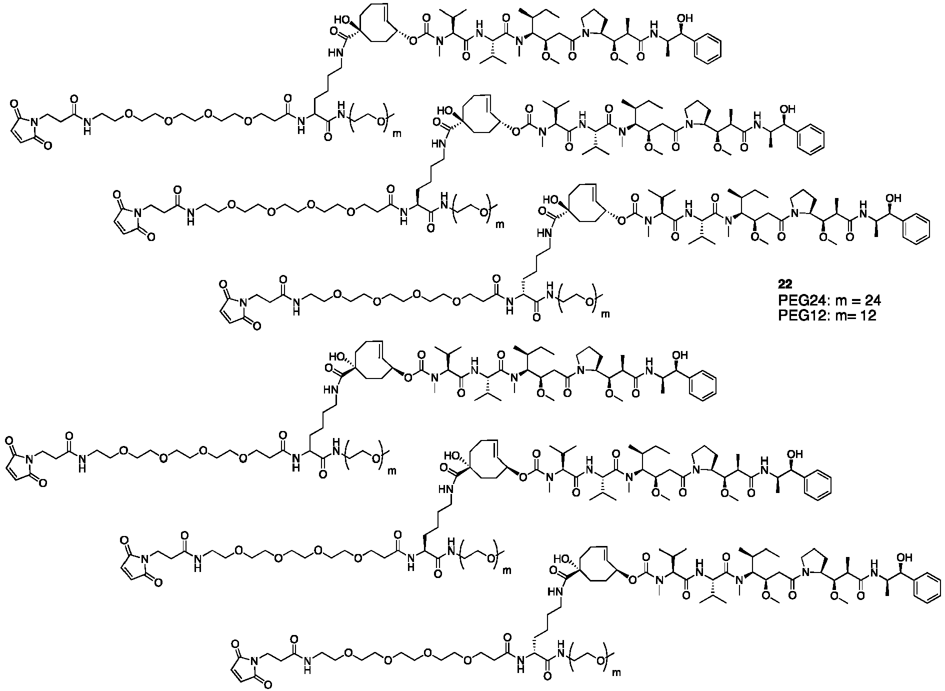

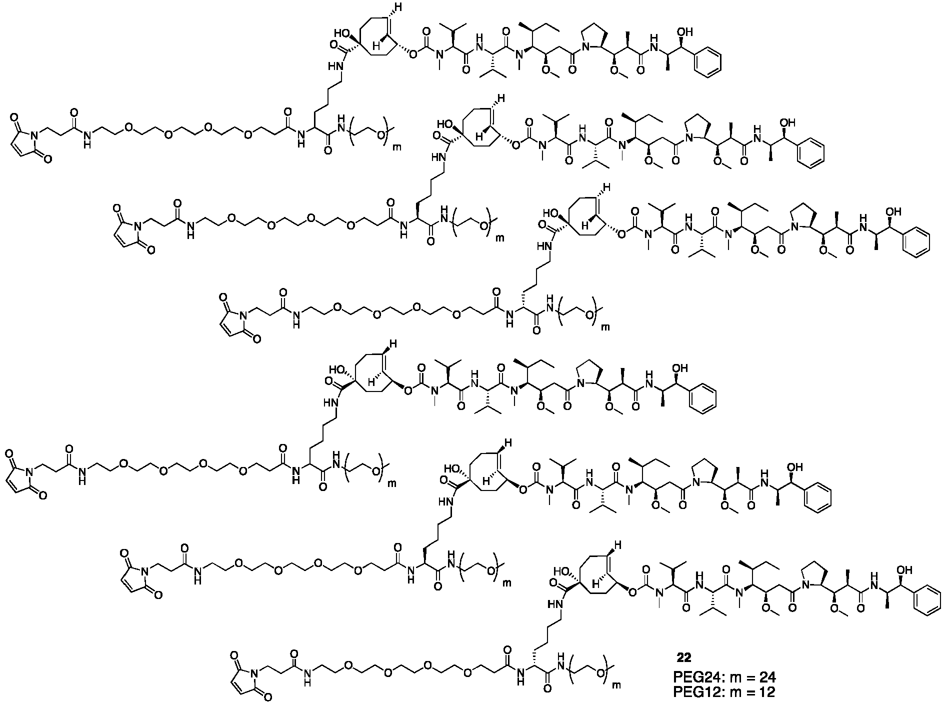

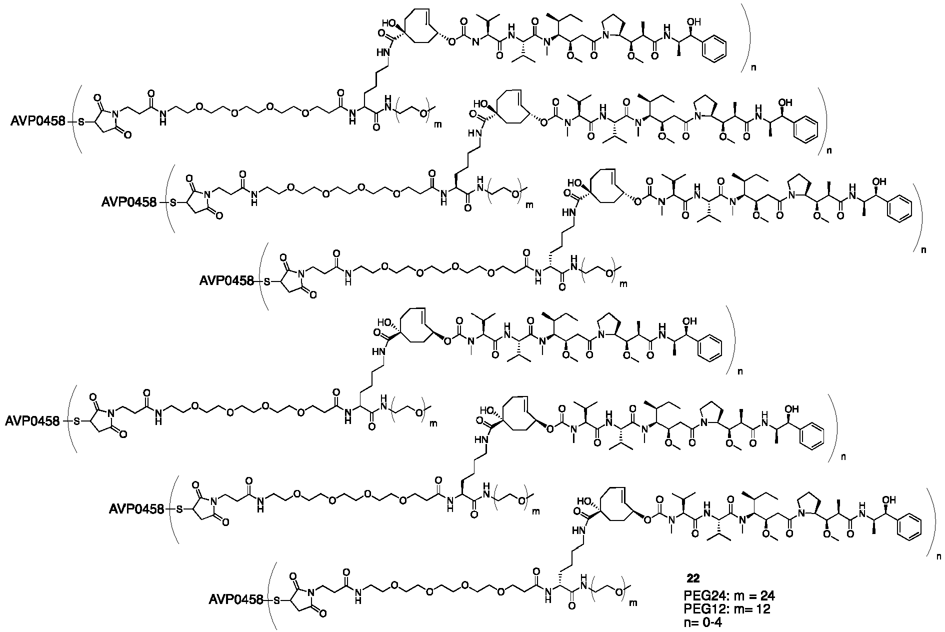

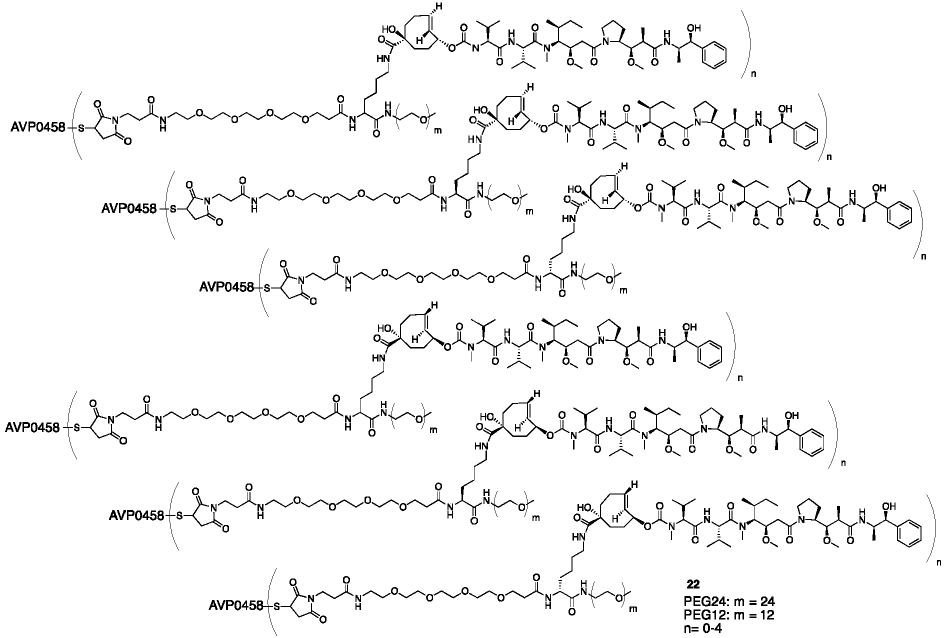

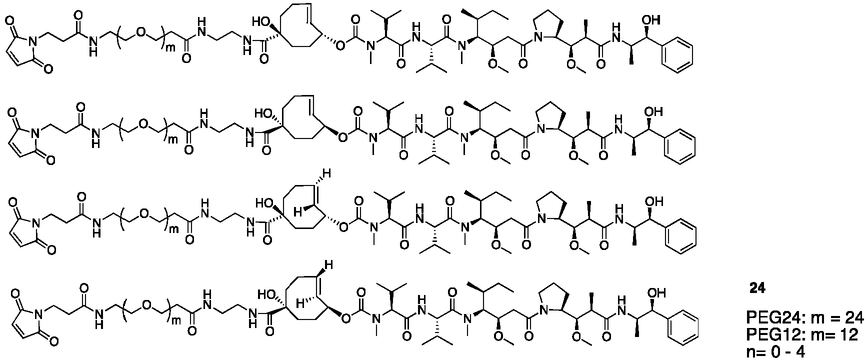

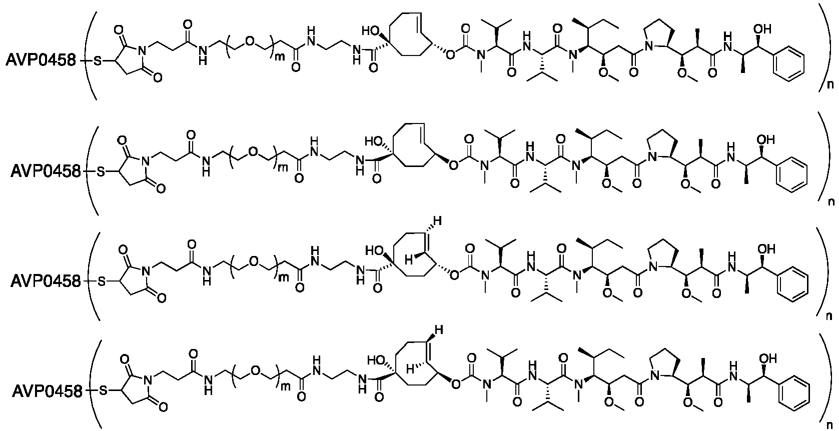

## Claims

1. A method for regioselectively modifying a compound according to Formula (3) at the R² position, wherein said method comprises the step of:
contacting said compound according to Formula (3) or a salt thereof with a compound comprising a secondary amine;
wherein Formula (3) is: 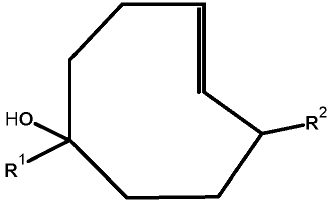
, wherein R¹ is selected from the group consisting of -C(O)O-N-succinimidyl, -C(O)O-pentafluorophenyl, -C(O)O-tetrafluorophenyl, -C(O)O-4-nitrophenyl, and -C(O)Cl; and
wherein R² is selected from the group consisting of -OC(O)O-N-succinimidyl, -OC(O)O-pentafluorophenyl, -OC(O)O-tetrafluorophenyl, -OC(O)O-4-nitrophenyl, and -OC(O)Cl.

2. The method according to claim 1, wherein the compound comprising a secondary amine is monomethyl auristatin E.

3. The method according to any one of the preceding claims, wherein the compound of Formula (3) is provided as a compound of Formula (3a) or as a compound of Formula (3b), or a mixture thereof, preferably a racemic mixture:

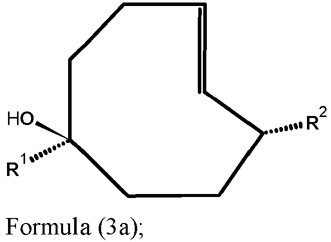

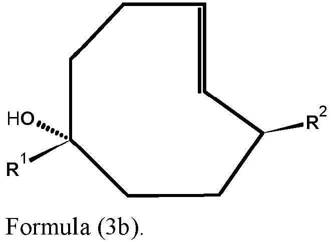

4. The method according to any one of the preceding claims, wherein R¹ is - C(O)O-N-succinimidyl, and wherein R² is -OC(O)O-*N*-succinimidyl.

5. The method according to any one of claims 1 to 3, wherein R¹ is -C(O)O-pentafluorophenyl, and wherein R² is -OC(O)O-pentafluorophenyl.

6. The method according to any one of the preceding claims, wherein the regioselective modification is carried out at a temperature in the range of from -80°C to 10 °C, preferably in the range of from -40°C to 5 °C, more preferably in a range of from -20°C to 0 °C, most preferably in a range of from -15°C to -5 °C.

7. The method according to any one of claims 1 to 5, wherein the regioselective modification is carried out at a temperature in a range of from 10°C to 40 °C, preferably in a range of from 15°C to 30 °C, and most preferably in a range of from 20°C to 25 °C.

8. The method according to any one of the preceding claims, wherein the method comprises the steps of:
a) dissolving said compound according to Formula (3) or a salt thereof, to form a solution;
b) contacting said solution with a compound comprising a secondary amine.

## Patentansprüche

1. Verfahren zum regioselektiven Modifizieren einer Verbindung der Formel (3) an der R²-Position, wobei das Verfahren den Schritt umfasst von:
Inkontaktbringen der Verbindung der Formel (3) oder eines Salzes davon mit einer Verbindung, umfassend ein sekundäres Amin;
wobei die Formel (3) ist: 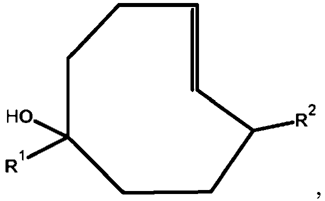
wobei R¹ ausgewählt ist aus der Gruppe bestehend aus -C(O)O-N-Succinimidyl, -C(O)O-Pentafluorphenyl, -C(O)O-Tetrafluorphenyl, -C(O)O-4-Nitrophenyl, und -C(O)Cl; und wobei R² ausgewählt ist aus der Gruppe bestehend aus -OC(O)O-N-Succinimidyl, -OC(O)O-Pentafluorphenyl, - OC(O)O-Tetrafluorphenyl, -OC(O)O-4-Nitrophenyl, und -OC(O)O-Cl.

2. Verfahren nach Anspruch 1, wobei die Verbindung, umfassend ein sekundäres Amin, Monomethyl-Auristatin E ist.

3. Verfahren nach einem der vorstehenden Ansprüche, wobei die Verbindung der Formel (3) als Verbindung der Formel (3a) oder als Verbindung der Formel (3b) oder als Mischung davon bereitgestellt wird, vorzugsweise als racemische Mischung:

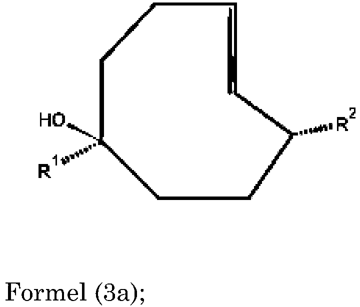

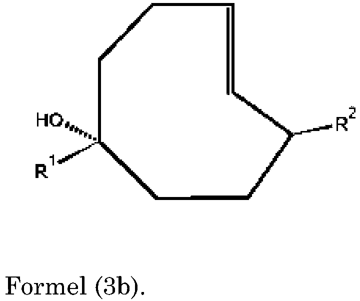

4. Verfahren nach einem der vorstehenden Ansprüche, wobei R¹ -C(O)O-N-Succinimidyl ist, und wobei R² -OC(O)O-N-Succinimidyl ist.

5. Verfahren nach einem der Ansprüche 1 bis 3, wobei R¹ -C(O)O-Pentafluorphenyl ist, und wobei R² -OC(O)O-Pentafluorphenyl ist.

6. Verfahren nach einem der vorstehenden Ansprüche, wobei die regioselektive Modifizierung bei einer Temperatur im Bereich von -80 °C bis 10 °C ausgeführt wird, vorzugsweise im Bereich von von -40 °C bis 5 °C, bevorzugter im Bereich von von -20 °C bis 0 °C, am bevorzugtesten im Bereich von von -15 °C bis -5 °C.

7. Verfahren nach einem der Ansprüche 1 bis 5, wobei die regioselektive Modifizierung bei einer Temperatur im Bereich von 10 °C bis 40 °C ausgeführt wird, vorzugsweise im Bereich von von 15 °C bis 30 °C und am bevorzugtesten im Bereich von von 20 °C bis 25 °C.

8. Verfahren nach einem der vorstehenden Ansprüche, wobei das Verfahren die Schritte umfasst von:
a) Auflösen der Verbindung gemäß Formel (3) oder eines Salzes davon, um eine Lösung zu bilden;
b) Inkontaktbringen der Lösung mit einer Verbindung, umfassend ein sekundäres Amin.

## Revendications

1. Procédé pour modifier de manière régiosélective un composé de formule (3) à la position R², dans lequel ledit procédé comprend l'étape de :
mise en contact dudit composé de formule (3) ou d'un sel de celui-ci avec un composé comprenant une amine secondaire,
dans lequel la formule (3) est : 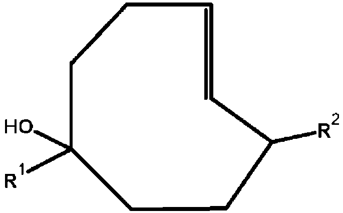
dans lequel R¹ est choisi dans le groupe constitué par -C(O)ON-succinimidyle, -C(O)O-pentafluorophényle, -C(O)O-tétrafluorophényle, -C(O)O-4-nitrophényle et -C(O)Cl ; et
dans lequel R² est choisi dans le groupe constitué par -OC(O)ON-succinimidyle, -OC(O)O-pentafluorophényle, -OC(O)O-tétrafluorophényle, -OC(O)O-4-nitrophényle et -OC(O)Cl.

2. Procédé selon la revendication 1, dans lequel le composé comprenant une amine secondaire est la monométhyl-auristatine E.

3. Procédé selon l'une quelconque des revendications précédentes, dans lequel le composé de formule (3) est fourni sous la forme d'un composé de formule (3a) ou d'un composé de formule (3b), ou d'un mélange de ceux-ci, de préférence d'un mélange racémique :

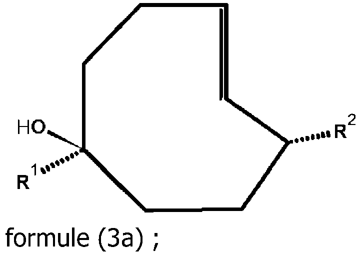

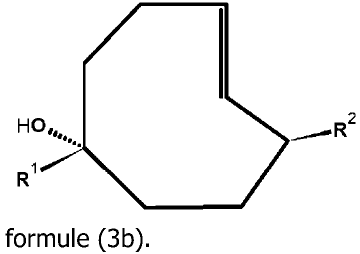

4. Procédé selon l'une quelconque des revendications précédentes, dans lequel R¹ est C(O)O-*N*-succinimidyle, et dans lequel R² est -OC(O)O-*N*-succinimidyle.

5. Procédé selon l'une quelconque des revendications 1 à 3, dans lequel R¹ est -C(O)O-pentafluorophényle, et dans lequel R² est -OC(O)O-pentafluorophényle.

6. Procédé selon l'une quelconque des revendications précédentes, dans lequel la modification régiosélective est effectuée à une température située dans la plage allant de -80 °C à 10 °C, de préférence dans la plage allant de -40 °C à 5 °C, mieux encore dans la plage allant de -20 °C à 0 °C, tout spécialement dans la plage allant de -15 °C à -5 °C.

7. Procédé selon l'une quelconque des revendications 1 à 5, dans lequel la modification régiosélective est effectuée à une température située dans la plage allant de 10 °C à 40 °C, de préférence dans la plage allant de 15 °C à 30 °C, et tout spécialement dans la plage allant de 20 °C à 25 °C.

8. Procédé selon l'une quelconque des revendications précédentes, dans lequel le procédé comprend les étapes de :
a) dissolution dudit composé de formule (3) ou d'un sel de celui-ci, pour former une solution ;
b) mise en contact de ladite solution avec un composé comprenant une amine secondaire.
